(19)

**Europäisches Patentamt**
**European Patent Office**
**Office européen des brevets**

(11) **EP 4 241 081 B1**

(12) **EUROPEAN PATENT SPECIFICATION**

(45) Date of publication and mention
of the grant of the patent:
**28.08.2024 Bulletin 2024/35**

(21) Application number: **21802748.0**

(22) Date of filing: **05.11.2021**

(51) International Patent Classification (IPC):
**G01N 33/542** (2006.01)     **C12Q 1/6818** (2018.01)

(52) Cooperative Patent Classification (CPC):
**G01N 33/542**

(86) International application number:
**PCT/EP2021/080827**

(87) International publication number:
**WO 2022/096677 (12.05.2022 Gazette 2022/19)**

(54) **ACCURATE BULK FRET**

GENAUER BULK FRET

FRET D'ENSEMBLE PRÉCIS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.11.2020 EP 20206272**

(43) Date of publication of application:
**13.09.2023 Bulletin 2023/37**

(73) Proprietor: **NanoTemper Technologies GmbH
81379 München (DE)**

(72) Inventors:
• **LÜDECKE, Annemarie
01309 Dresden (DE)**
• **OSSEFORTH, Christian
86919 Utting am Ammersee (DE)**
• **BAASKE, Philipp
81379 München (DE)**
• **MOYA MUNZO GUSTAVO, Gabriel
81377 Munich (DE)**
• **CORDES, Thorben
82131 Gauting (DE)**
• **GEBHARDT, Christian
81373 Munich (DE)**

(74) Representative: **Vossius & Partner
Patentanwälte Rechtsanwälte mbB
Siebertstraße 3
81675 München (DE)**

(56) References cited:
**US-A9- 2019 353 663**

• **MAJUMDAR ET AL: "Measurements of Internal
Distance Changes of the 30S Ribosome Using
FRET with Multiple Donor-Acceptor Pairs:
Quantitative Spectroscopic Methods", JOURNAL
OF MOLECULAR BIOLOGY, ACADEMIC PRESS,
UNITED KINGDOM, vol. 351, no. 5, 2 September
2005 (2005-09-02), pages 1123 - 1145,
XP005022198, ISSN: 0022-2836, DOI:
10.1016/J.JMB.2005.06.027**
• **ALEXIS COULLOMB ET AL: "QuanTI-FRET: a
framework for quantitative FRET measurements
in living cells", ARXIV.ORG, CORNELL
UNIVERSITY LIBRARY, 201 OLIN LIBRARY
CORNELL UNIVERSITY ITHACA, NY 14853, 17
December 2019 (2019-12-17), XP081560124**

## Description

### Field of Invention

The present invention relates to a method for obtaining quantitative information on average donor-acceptor distance changes within a molecule, between molecules or within/between molecule complexes using ensemble Förster Resonance Energy Transfer (eFRET; in the following also called quantumFRET), and a measurement system comprising a controller adapted for performing the same. The method comprises the steps of performing an eFRET measurement for at least a first sample with a first fluorescent dye, preferably a donor-labelled sample, a second sample with a second fluorescent dye, preferably an acceptor-labelled sample, and a third sample comprising both dyes, preferably a donor- and an acceptor-labelled sample. Preferably, each sample comprises respective labelled molecule copies and wherein each eFRET measurement is preferably performed using multiple respective labelled molecule copies under a first and a second condition. In case each sample comprises labelled molecule copies, it is preferred to correct the obtained results for condition-specific and/or inter-condition effects and to determine condition-specific eFRET efficiencies based on the corrected results. Quantitative information on donor-acceptor distance changes are then determined within the molecule or between molecules between the first and the second condition based on the respective eFRET efficiencies.

In particular, the method and the system of the present invention provide a quantitative high-throughput measurement of distance or distance change within or between biomolecules, especially during changes in the environment which include but are not limited to changes in pH, ionic strength, temperature, addition of chaotropes, buffer composition, addition of ligands, addition of binding partners, addition of detergents or combinations thereof.

### Background

Förster or fluorescence resonance energy transfer (FRET), first described by Theodor Förster in 1946, is a physical phenomenon in which a donor fluorophore in its excited state non-radiatively transfers its excitation energy to an acceptor fluorophore that is located in close proximity to the donor, thereby causing the acceptor to emit its fluorescence. Since FRET is highly sensitive to the distance between donor and acceptor, FRET-based donor acceptor pairs can be used as biosensors and especially as spectroscopic rulers to monitor a variety of biochemical activities that produce changes in molecular distances, such as protein-protein interactions, conformational changes, intracellular ion concentrations, and enzyme activities.

FRET can occur between two fluorophores that are in close proximity to each other and that have a substantial overlap, e.g. at least 30%, between the donor's emission and acceptor's absorption spectra. FRET is characterized by the FRET efficiency ($E$, in the latter also denotes as accurate FRET efficiency, $E_{real}$), which refers to the percent of energy transfer from the donor to acceptor at a given state and can quantitatively described as follows:

$$E_{real} = \frac{1}{1+\frac{r^6}{R_0^6}} = \frac{R_0^6}{r^6 + R_0^6} \qquad \text{(I)},$$

wherein r denotes the distance between donor and acceptor dipoles, and $R_0$ the distance at which FRET $E$ is 50%. As the FRET efficiency decreases at the sixth power as the distance between donor and acceptor increases, FRET is extremely sensitive to small changes in distance, e.g. with a distance range of 1-10 nm and spatial resolution in the range of a few Angstrom that can even be observed in real time. This has made FRET interesting for different research areas, including chemistry, biochemistry, molecular biology and cell biology, and allows even the investigation of target molecules in living cells, i.e. in their physiological context.

FRET-based biosensors can be classified into intermolecular biosensors, in which donor and acceptor are fused to different molecules, and FRET changes occur when the independent molecules come into close proximity, and into intramolecular biosensors, in which donor and acceptor are conjoined to the same molecule and in which conformational changes in the molecule induce FRET changes.

By ligating for example an acceptor to a first molecule and a donor to a second molecule, FRET can be used for detecting co-localization and/or interactions between said molecules and, ideally, even for estimating the distance between them. Further examples of FRET applications include the detection of interaction, homodimerization, heterodimerization, and/or oligomerization of proteins, and the elucidation of signal transduction pathways. FRET can even be used to investigate protein-protein, protein-nucleic acid and nucleic acid-nucleic acid interactions for example and can also be combined with other techniques such as fluorescence or confocal microscopy.

Alternatively, donor and acceptor can be localized in the same molecule, for example by hybridization or ligation. Thus, FRET can be used, e.g., to quantify nucleic acids using real-time quantitative PCR by adding donor and acceptor labelled molecules that bind specifically to the same target DNA during a PCR cycle, wherein binding of said labelled molecules to the DNA in close proximity to each other enables FRET with the FRET signal increasing as a function of the target DNA concentration. Furthermore, information about conformational changes can be obtained using intramolecular FRET-based biosensors. As a change in the conformation of a molecule such as a protein, e.g. through association with other proteins, binding of a ligand and/or catalytic activity, is associated with a change in certain interatomic distances within said protein, such changes can be tracked by labeling the involved structural units with one or more donor-acceptor pairs. With the new method and system of the present invention, binding domains can be investigated and the effects of (new) substrates for different enzymes or potential receptor agonists evaluated quantitatively and in high throughput, e.g. in the context of drug discovery.

As biochemical activities that produce changes in molecular distances are directly correlated with FRET efficiency, methods have been developed to measure the FRET efficiency change in various ways, including changes in fluorescence intensity of donor fluorophores, fluorescence intensity change of acceptor fluorophores, lifetime of donor fluorescence or a combination thereof. However, results of FRET measurements can vary substantially. Reasons include, e.g., the presence of false-positive signals of relatively high intensity due to the required superimposition of the emission and excitation spectrum of donor and acceptor. As the emitted fluorescence after the energy transfer is often comparatively weak, an unfavorable signal-to-noise ratio can impede analyses. Furthermore, the degree of labeling can vary substantially between experiments and consequently also the ratio of false positives to the experimentally desired signal.

In the prior art, FRET is mostly used in a single molecule context for precise distance quantification, usually in academia, and in ensemble measurements only for qualitative results, both in industry and academia. In prior art FRET based ensemble assays that are used to monitor interactions, both the ligand and binding partner are labelled, and binding is typically assessed only qualitatively.

In single molecule FRET (smFRET) experiments, several corrections are applied to the raw data to yield accurate distance information, but measurements require surface immobilization or long measurement durations. Single molecule FRET experiments also require very expensive setups, in a special LASER lab and only very specialized experts can use it. Thus, these kinds of experiments can typically only be used in special labs at universities and are not attractive or usable for industry.

For instance, single molecule FRET (smFRET) is typically based on the analysis of one biomolecule at a time using a highly diluted liquid solution comprising the molecule under study, e.g. with the concentration of said molecule being in a picomolar range. Due to the low concentration of the molecule it can be ensured that one molecule passes the focus of a laser of a modified confocal microscope. For example, the modified confocal microscope can comprise a laser, the light of which is focused using an objective lens and a high numerical aperture to generate a diffraction limited excitation spot in a very small sample volume comprising the molecule under study such as a small sample volume in the range of a femto-liter. The signal emitted from the molecule under study diffusing through the laser focus one by one due to diffusion can then be collected by the objective lens, filtered for background signal using a pinhole for example, and split into respective donor and acceptor light detector channels using for example a dichromatic mirror. However, as smFRET relies on diffusion-based measurements, smFRET analyses can be time consuming with a single experiment for a molecule under study in one conformation requiring for example up to 30 minutes. As smFRET approaches are quite time consuming, the use of smFRET has been limited for high throughput applications in industry for example. The alternatively used TIRF-FRET which also has single molecule capabilities, suffers from limitations regarding surface-immobilization of molecules.

Moreover, other methods for detection of conformational changes are, for example, X-Ray and Cryo-EM (cryogenic electron microscopy), which have a low throughput, are expensive and need a very complex and long sample preparation.

Hence, there is still a need to have at hand alternative solutions for distance or distance change measurement at a molecular level in high throughput based on accurate quantitative intra- and/or intermolecular distance measurements by FRET.

Summary of the Invention

The invention is defined in the appended claims.

In general, according to the present invention, the distance or distance change can be determined through energy transfer between two fluorescent dyes (fluorophores), proteins, in general substances emitting fluorescent light when excited fluorescently, which are attached to the molecule(s) of interest, by using energy transfer between two or more fluorophores, Förster Resonance Energy Transfer ("FRET"). The efficiency of the energy transfer contains information about the distance of at least a fluorophore pair. The energy transfer efficiency of the present invention is preferably based on a fluorescently labeled entity, which can be a protein, DNA or any other (bio)molecule or supramolecular complex. For the sake of clarity, it is simply called molecule or biomolecule. The quantitative outcome is the energy transfer efficiency of a biomolecule or the ratio of two energy transfer efficiencies of the same biomolecule in two potentially different conformational states, e.g. with and without binding partner or with two different binding partners. For the second case, given a sample with one known energy transfer efficiency, using the energy transfer efficiency ratio, the unknown energy transfer efficiency can be calculated.

Having the additional knowledge of the Förster radius of a fluorophore pair, one can calculate inter-fluorophore distances by measuring the energy transfer efficiency. In the case of measuring the energy transfer efficiency ratio, the additional information of the energy transfer efficiency or distance of one of the conformational states can be used such that quantitative distances between the fluorophore pair in the different conformations can be calculated.

Moreover, having knowledge of the Förster radius of a fluorophore pair, having knowledge of the 3D structure of the biomolecule labelled with the fluorophore pair and having knowledge of the labelling positions of the fluorophores at the 3D structure of the biomolecule the ligand induced distance change between the fluorophores, and thus the change in the 3D structure of the labelled biomolecule can be quantified, preferably with nanometer resolution, more preferably with Angström resolution.

By using the method of the present invention, one can significantly accelerate ligand screening for binding partners that do or do not lead to a change in conformation. Especially since the quantitative nature of the result allows for ranking of ligand-induced distance/distance change in screenings.

In other words, the present invention is particularly based on the finding that a quantitative ensemble measurement of FRET efficiencies can be obtained for a labelled molecule, e.g. a protein, under two conditions, e.g. with and without binding partner or with two different binding partners. This has the advantage that, given a molecule with one known FRET efficiency under one of the two conditions, the unknown second FRET efficiency under the other condition can be calculated based on the FRET efficiency ratio obtained by ensemble Förster resonance energy transfer (eFRET). Moreover, if the distance of the donor-acceptor pair in one conformation and the Förster Radius of said pair is known, distances between said donor and acceptor can be calculated under other conditions and thus, for other conformations using the eFRET efficiency ratio of the present invention.

Another approach is to group different binding partners by the conformational change they evoke. For this approach one does not have to have any prior knowledge about the protein structure or the exact distance between the labeling sites in a certain condition and can still judge if a ligand binds using the same mechanism as another ligand. This is, for example, an important question in finding allosteric binders, which are hard to screen for.

The present invention provides a plurality of advantages over the prior art. For instance, with methods and systems of the present invention, it is possible to measure conformational changes of biomolecules with angstrom resolution in free solution in an easy to use and economic way. For instance, known prior art ensemble FRET measurements only yield quantitative data after using several correction methods. So far ensemble FRET measurements have been corrected for background and the measurement artifacts of so-called "bleed-through" of excitation and emission light. Another vital correction, gamma-correction, which corrects for the different observed brightness of donor and acceptor fluorophore has been addressed with different approaches amongst them using (1) a reference compound with a pre-defined FRET efficiency, (2) acceptor photobleaching and (3) the use of reference compounds with different, but not necessarily known, FRET efficiencies.

According to the present invention, it is preferred to use the (2) acceptor photobleaching and/or a new approach, e.g., (4) ligand induced energy transfer efficiency change, in which the biomolecule can be used as its own reference compound.

Moreover, the inventors of the present invention also noted that there is a remaining issue in ensemble FRET measurements which is related to the presence of incompletely labeled biomolecules, especially donor-only labeled ones. These molecules may skew the measured FRET efficiency with a factor depending on labeling ratio. Alternatively to (a) the removal of donor-only labeled molecules and (b) correcting the FRET efficiency using measured concentrations, the present invention preferably proposes another new approach, e.g., (c) to take the ratio of skewed FRET efficiencies, which is identical to the unskewed FRET efficiency ratio and much less prone to measurement inaccuracies.

Moreover, as both absorbance cross section as well as quantum yield are typically environment and temperature sensitive, measurements according to the present invention are preferably conducted at (at least) one predetermined temperature and preferably under environment control.

Basic ideas of the present invention may be summarized as follows. The present invention preferably comprises a simple labeling and measurement routine that preferably encompasses at least one of: (1) background correction, (2) leakage correction for the donor emission erroneously quantified as acceptor emission (see e.g. $c_{lk}$), (3) direct excitation correction for acceptor molecules that are directly excited by the donor excitation light (see e.g. $c_{dirE}$), (4) gamma ($\gamma$) correction which corrects for differences in observed donor and acceptor brightness stemming from differences in quantum yield and detection efficiencies, (5) a way to mitigate the effects of incomplete labeling on the measured FRET efficiency as well as (6) temperature control. These corrections allow for quantitative FRET efficiency measurements in an ensemble of incompletely labeled molecules.

Additionally, the method of the present invention provides a clear set of instructions for labeling and measurements, but also a set of shortcuts that may be suitable for certain dyes or dye combinations.

General ideas of the present invention may be summarized as follows:
If no appropriate intrinsic fluorescence donor/acceptor pair is present in the biomolecule, then the biomolecule is preferably labelled with either a so-called donor fluorophore or a so-called acceptor fluorophore, or both, where the distance between the so-called acceptor and so-called donor should be in the range for FRET to occur. Preferably, three fluorogenic samples are then prepared. The third one is preferably the biomolecule labeled with both a so-called donor fluorophore and a so-called acceptor. The first and second samples preferably comprise a so-called donor fluorophore and a so-called acceptor fluorophore, respectively, which are either measured as free fluorophores or functionalized to a biomolecule, preferably the biomolecule in question.

The three fluorogenic samples are then measured, preferably at a predetermined temperature. Preferably it can also be measured at two different temperatures and that the binding induced conformational change is characterized based on the measurements at these different temperatures.

The two different temperatures can be at the same time but at different places, for example in a temperature gradient, and/or at different times and different places or at different times at the same place/sample.

The apparent FRET efficiency of the double-labeled sample can be calculated using correction factors obtained from the measurement of all three fluorogenic samples. The real, accurate FRET efficiency $E_{real}$ and the apparent FRET efficiency after corrections $E_{app}$ are related in the following way:

$$E_{app} = E_{real} \frac{c_{DA}}{c_{DA} + c_D}$$

$c_{DA}$ and $c_D$ are the concentrations of the double-labeled biomolecule and the donor only labeled biomolecule, respectively. It is important to note that labeling is usually not complete meaning that there will usually be donor only labeled biomolecule within the double labeled biomolecule sample.

This shows, for instance, that the apparent FRET efficiency depends on the labeling success of the double-labeled sample. This dependency can be removed either by quantifying the concentrations or the concentration ratio of correctly double-labeled biomolecule and erroneously labeled biomolecules which only carry a donor-fluorophore or by an added purification step that removes erroneously donor-only labeled biomolecules from the double-labeled sample or preferably by quantifying the ratio of FRET efficiencies of two different biomolecule states, such with and without a bound ligand.

The absolute FRET efficiency can be as precise as the concentration measurement or as accurate as the additional purification. The ratio of FRET efficiencies from a bulk or ensemble measurement can be as precise as single molecule measurements.

$$\frac{E_{app1}}{E_{app2}} = \frac{E_{real1} \frac{c_{DA}}{c_{DA} + c_D}}{E_{real2} \frac{c_{DA}}{c_{DA} + c_D}} = \frac{E_{real1}}{E_{real2}}$$

According to a preferred embodiment, the present invention relates to a method for obtaining quantitative information on average donor-acceptor distance changes within a molecule using ensemble Förster resonance energy transfer (eFRET), the method comprising the steps of: obtaining or providing a donor sample, said donor sample comprises at least a donor and/or donor-labelled copies of said molecule, an acceptor sample, said acceptor sample comprises at least an acceptor and/or acceptor-labelled copies of said molecule, and a donor-acceptor-labelled sample comprising donor-acceptor-labelled copies of said molecule. Ensemble FRET (eFRET) measurements are performed for the donor-acceptor-labelled sample under a first and a second condition to obtain for this sample a first eFRET result under the first condition and a second eFRET result under the second condition. Ensemble FRET measurements are preferably also performed for the donor sample and the acceptor sample under at least one condition, preferably under at least one of the first and second conditions, preferably under both of said first and second condition. Thus, at least four, preferably six measurements are performed.

Ensemble FRET measurements for the donor sample and/or the acceptor sample may alternatively be performed under other conditions which are neither the first nor second conditions. For instance, in case the donor-labels and/or acceptor labels are relatively insensitive on changes of conditions, ensemble FRET measurements of a third condition and/or a fourth condition may be used. For instance, such third and/or fourth conditions for acceptor and/or donor ensemble FRET measurements may be performed in advance of the above-mentioned measurements, e.g., under specific predetermined conditions, wherein the measurements of these other conditions may be stored in the system such that these stored values may be used as reference values instead of performing the ensemble FRET measurements for the donor sample and/or the acceptor sample. In other words, only two measurements for the donor-acceptor-labelled sample under the first and second condition are necessary.

Moreover, obtaining a sample within the sense of the present application relates to getting or receiving a sample outside a human or animal body. In other words, the obtaining step does not cover any surgery method practiced on a human or animal body. Instead of obtaining a sample before the measurement steps, providing the respective sample before the measurement steps can be synonymously used.

The eFRET measurement of the donor-acceptor-labelled sample is performed using multiple respective molecule copies. The obtained first and second eFRET results are preferably corrected, e.g. by considering leakage effects, direct excitation effects and/or gamma corrections. These effects are typically fluorophore-specific. In case also the first and second samples comprise labelled molecules, the corrections may also be grouped in condition-specific and inter-condition effects. In particular, condition-specific or fluorophore-specific eFRET effects are preferably determined on the basis of the donor sample and the acceptor sample, wherein inter-condition effects are preferably determined on the basis of the donor-acceptor-sample. Quantitative information can be determined on average donor-acceptor distance changes within the molecule under the first and the second condition using the condition-specific eFRET efficiencies. Similar steps are performed if quantitative information on average donor-acceptor distance changes between a first and second molecule is determined.

Preferably, the step of obtaining at least the donor-acceptor-labelled sample, and optionally the acceptor sample with acceptor-labelled copies of said molecule and the donor sample with donor-labelled copies of said molecule comprises the steps of: obtaining at least a first, a second, and a third sample comprising multiple copies of the molecule each; labelling the molecule copies comprised in the first sample with the donor; labelling the molecule copies comprised in the second sample with the acceptor; and labelling the molecule copies comprised in the third sample with the donor and the acceptor.

Preferably, the donor is a fluorophore and wherein the acceptor is preferably another fluorophore. Optionally or alternatively, it is preferred that the donor has a fluorescence emission spectrum and the acceptor an excitation spectrum, and wherein the fluorescence emission spectrum of the donor overlaps with the excitation spectrum of the acceptor. In other words, the emission wavelength of the so-called donor fluorophore should overlap with the excitation spectrum of the so-called acceptor fluorophore and the two fluorophores should have different excitation and emission wavelengths. FRET can preferably be measured at room temperature and preferably in aqueous solutions in the native state of the biomolecule (e.g. no crystallization is necessary).

Preferably, the step of performing an eFRET measurement for each of said samples under each condition comprises for each eFRET measurement the steps of: transmitting light having a wavelength within the excitation spectrum of the donor, preferably from a monochromatic light source, preferably an LED, to the respective sample to excite the donor, and transmitting light having a wavelength within the excitation spectrum of the acceptor, preferably from a monochromatic light source, preferably an LED, to the respective sample to excite the acceptor. Optionally, it is preferred that the light is transmitted to the respective sample in at least one illumination cycle, preferably 1-500 illumination cycles, wherein an illumination cycle has at least one, preferably 2 light pulses or more in the range between 10 ms and 1s sec, preferably light pulses with light having alternately a wavelength within the excitation spectrum of the donor and the acceptor, respectively.

Preferably, the step of performing an eFRET measurement for each of said samples under the first and the second condition comprises for each eFRET measurement the step of: if the acceptor is a fluorophore: measuring the emission of the acceptor after excitation with two different wavelengths, preferably the fluorescence emission of the acceptor, and measuring the emission of the donor, preferably the fluorescence emission of the donor. Optionally, it is preferred that the measured emissions of the acceptor are averaged and/or the measurement emissions of the donor are averaged. Optionally, the measured emission of the acceptor and the donor, respectively, is averaged over the at least one illumination cycle.

Preferably, the first and the second eFRET results each comprise respective measurement values of the emission of the acceptor after donor excitation ($F_{DA}$), emission of the acceptor after acceptor excitation ($F_{AA}$), and/or emission of the donor after donor excitation ($F_{DD}$). Optionally, it is preferred that respective measurement values are obtained of $F_{DA}$ for the donor sample, the acceptor sample, and the donor-acceptor-labelled sample, $F_{AA}$ for the acceptor- and the donor-acceptor-labelled sample, and $F_{DD}$ for the donor- and the donor-acceptor-labelled sample.

Preferably, wherein the first and the second condition differ from each other i) by the presence of a further molecule in one of the two conditions, wherein the further molecule preferably interacts with the molecule, and/or ii) in at least one, preferably one, parameter selected from the group consisting of temperature, pH, salt content, buffer composition, addition of chaotropes , excipients and ionic strength.

The step of correcting the obtained first and second eFRET results comprises the steps of determining fluorophore-specific correction factors and/or condition-specific correction factors, e.g. using the respective first and second eFRET results. For instance, the first and second eFRET results are preferably corrected using the respective condition-specific correction factors, and preferably an inter-condition correction factor is determined by using the condition-specific corrected first and second eFRET results. Based on the donor sample and the acceptor sample which may comprise corresponding copies of donor-labelled molecules and acceptor labelled molecules, respectively, condition-specific corrected first and second eFRET results can be obtained.

In case pure fluorophores are used instead of the donor-labelled or acceptor labelled copies, fluorophore-specific correction factors are obtainable. Moreover, the present invention preferably also uses the inter-condition correction factor. According to the present invention that the step of determining correction factors using the respective first and second eFRET result comprises the steps of: determining a leakage coefficient $c_{lk}$, and/or determining a direct excitation coefficient $c_{dirE}$. $c_{lk}$ is determined based on the ratio of $F_{DA}$ and $F_{DD}$, wherein said $F_{DA}$ and $F_{DD}$ are preferably obtained for the donor sample under the respective condition, and/or $c_{dirE}$ is determined based on the ratio of $F_{DA}$ and $F_{AA}$, wherein said $F_{DA}$ and $F_{AA}$ are obtained for the acceptor sample under the respective condition.

Preferably, the step of correcting the first and second eFRET results using the respective correction factors comprises the step of determining a corrected $F_{DA}$ ($F_{DAcor}$) for the donor-acceptor-labelled sample by subtracting from $F_{DA}$ the $c_{lk}$ corrected $F_{DD}$ and the $c_{dirE}$ corrected $F_{AA}$, wherein said $F_{DA}$, $F_{DD}$, and $F_{AA}$ are measured for the donor-acceptor-labelled sample under the respective condition.

The step of determining an inter-condition correction factor using the corrected first and second eFRET results comprises the step of determining a $\gamma$-correction factor based on the negative ratio of i) the difference of $F_{DAcor}$ between the first and the second condition and ii) the difference of $F_{DD}$ of the donor-acceptor-labelled sample between the first and the second condition. Preferably, $F_{DD}$ and $F_{DAcor}$ are first normalized by the samples FAA, to mitigate effects of concentration difference.

Preferably, the step of determining eFRET efficiencies based on the respective corrected first and second eFRET result comprises the steps of: determining respective $\gamma$-corrected $F_{DD}$ for the donor-acceptor-labelled sample under the respective condition, and determining eFRET efficiencies based on the ratio of i) the respective $F_{DAcor}$ and ii) the sum of the respective $F_{DAcor}$ and the respective $\gamma$-corrected $FDD$.

Preferably, the step of determining quantitative information on average donor-acceptor distance changes within the molecule under the first and the second condition using the eFRET efficiencies comprises the step of obtaining information on a donor-acceptor specific Förster radius $R_0$ and on a donor-acceptor distance within the molecule under the first or the second condition r.

Preferably, the method is carried out by a computer.

The present invention relates further to a computer program comprising instructions which, when the program is executed by a computer, cause the computer to carry out the method according to the invention.

The present invention relates further to a computer-readable data carrier comprising instructions which, when executed by a computer, cause the computer to carry out the method according to the invention.

The present invention relates further to a measurement system comprising a controller adapted for obtaining quantitative information on average donor-acceptor distance changes within a molecule using ensemble Förster resonance energy transfer (eFRET), preferably in accordance with any of the above mentioned methods, wherein said measurement system further comprises: means for accommodating a vessel, preferably a microwell plate or a capillary, comprising a donor-labelled sample comprising donor-labelled copies of said molecule, an acceptor-labelled sample comprising acceptor-labelled copies of said molecule, and/or a donor-acceptor-labelled sample comprising donor-acceptor-labelled copies of said molecule; a monochromatic light source, preferably an LED or a superluminescent light emitting diode (SLED or SLD, which combines the high power and brightness of laser diodes with the low coherence of conventional light-emitting diodes.), and a light detector, preferably a photo multiplier tube (PMT), a charge-coupled device (CCD), a complementary metal oxide semiconductor (CMOS) or silicon photomultiplier (SiPM); preferably a filter, a dichronic mirror, a polychromic mirror, and/or an objective lens; means for performing an eFRET measurement using multiple respective molecule copies; and control means adapted for controlling the means for accommodating the vessel; controlling the monochromatic light source for transmitting light from the monochromatic light source to the vessel; controlling the light detector for detecting signals from the vessel; preferably controlling temperature; and controlling said means for performing the eFRET measurement.

The present invention relates further to the use of a measurement system of the present invention to obtain quantitative information on average donor-acceptor distance changes within a molecule using ensemble Förster resonance energy transfer (eFRET), preferably in accordance with the method of the invention.

The light may be directed to the vessel in form of a "free" beam of light which is directed by use of optical means, e.g., lenses, mirrors etc. Moreover, a person skilled in the art also appreciates that light fibers, e.g., polarizing maintaining light fibers or light fibers which do not maintain polarization may be used somewhere between the light source and the vessel and/or between the light source and the detector.

Preferably, the measurement comprises the steps of obtaining an analog output signal obtained from the light detector; and processing the obtained analog output signal. Preferably, the step of processing the obtained analog output signal comprises the step of digitalizing the obtained analog output signal into a digitalized output signal. Digitizing the (entire) analog output signal from the detector provides certain advantages over the prior art which will be discussed in further detail below. In particular, the resulting digitalized output data may be considered as a digitalized raw signal, which is, however, easier to handle subsequently depending on the intensity of the light detected by the detector. In short, the digitalized output data can be subsequently processed with analog process means or processed as by digital process means, e.g., to discriminate individual peaks for individual photons. Preferably, the step of processing the output signal further comprises the step(s) of i) processing the digitalized output signal as a digitalized single photon pulse signal, preferably in case the intensity of the detected light emitted from the vessel is below 2 million detected photons per second; and/or ii) processing the digitalized output signal as discrete values of an analog signal, preferably in case the intensity of the detected light is above 2 million detected photons per second. Preferably, the step of processing the obtained output signal comprises either step i) or step ii), and wherein the time to decide whether to process the digitalized output signal as a digitalized signal according to step i) is less than 1 sec, preferably maximal 0.05 sec, preferably by using an FPGA (Field Programmable Gate Array). Moreover, both signals, i.e., photon counting signal and discrete value signal, may be processed simultaneously. Like this, the decision whether to process according to step i) or ii) can be met after the measurement.

Preferably, the step of processing the obtained output signal further comprises the step(s) of storing the processed digitalized output signal obtained from step i) or step ii); or storing the processed digitalized output signals obtained from step i) and step ii); and further processing one of the stored output signals.

As an example how the signal is processed, if it is processed as a photon counting signal and/or an analog signal, is not pre-determined by the detector detecting the photon, for example as it would be when using a dedicated photon-counting detector, but at a later stage by algorithms in for example a FPGA (Field Programmable Gate Array), ASIC (application-specific integrated circuit) or other programmable means. Preferably these algorithms can be changed, for example improved, without changing the hardware. For example a limit of 2 Million photons at which the algorithm changes from flagging the signal as photon counting signal to flagging it as analog signal, can be updated and/or changed by firmware and/or software means. For example the limit can be switched from 2 Million detected photons per second to for example 1 Million detected photons per second or 4 Million detected photons per second. This is preferable with respect to existing solutions in which the hardware, for example the photon counter, has to be exchanged to alter the above mentioned limit.

Several experiments were conducted which illustrate that the measurement scheme of the present invention yields high quality quantitative data, comparable in quality to much more elaborate, costly and time-consuming state-of-the-art smFRET data and methods based on X-Ray or Cryo-EM, which have a much lower throughput, are much more expensive and need a very complex and long sample preparation.

Brief description of the drawings

In the following, preferred embodiments of the present invention are described in detail with respect to the Figures. The Figures show:

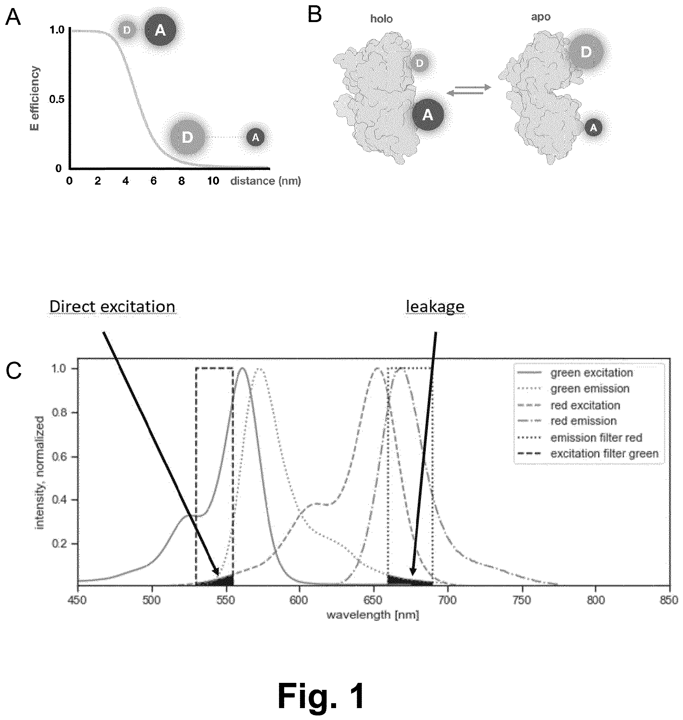

**Figure 1:**    FRET efficiency dependency on the distance between the dipoles.

(A) Sigmoidal curve describing the FRET efficiency dependency on the distance between donor (D) and acceptor (A).
(B) Conformations of molecules such as proteins can be quantitatively measured using eFRET according to the present invention. Exemplarily depicted is a protein in a first (left; closed conformation) and a second conformation (right; open conformation), respectively. Transitions of the conformations of the molecule can occur depending on a respective change of two conditions.
(C) Donor and acceptor absorption and emission spectra in case of Alexa546 (D) and Alexa647 (A). FRET occurs in the spectral conformation overlap between D emission and A absorption. Direct excitation (Dir) can occur in the absorption spectra overlap of D and A. Leakage (Lk) can occur in the emission spectra overlap between D and A. Two optional acquisition filters are depicted exemplarily.

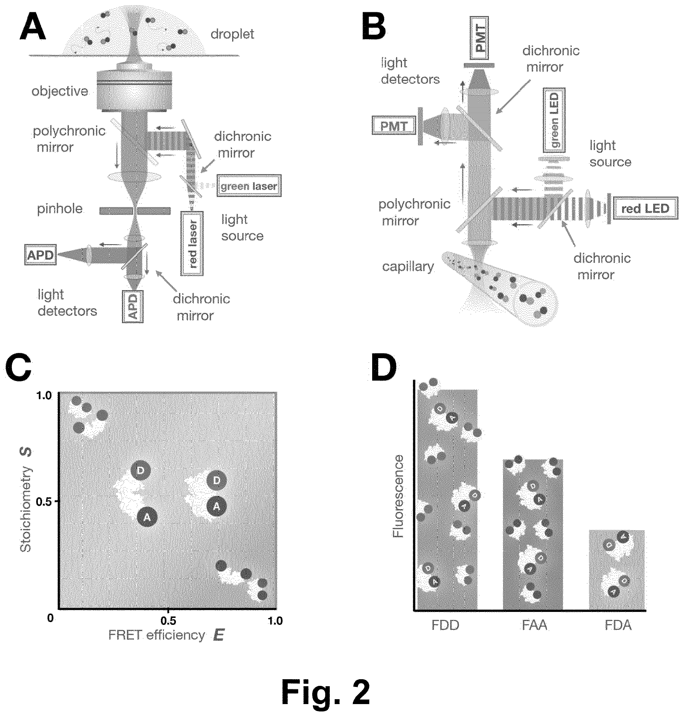

**Figure 2:**    Comparison of single-molecule (sm)FRET and eFRET (quantumFRET) setups.

(A) smFRET setup: The light source, here depicted as two lasers, emits alternating high-frequency pulses of red and green light, which are combined by a dichronic mirror, followed by the deflection of the light by a polychromic mirror into an objective with a high numerical aperture. The high numerical aperture is used to generate a diffraction limited excitation spot in the sample volume. Signals, e.g. fluorescence, originating from the molecule copies diffusing through the femto-liter excitation volume, are collected by said objective, and pass said polychromic mirror and an objective lens. Using a pinhole, the collected fluorescence signal is background-filtered, split into green and red fluorescence using a dichroic mirror, and then detected by a respective light detector, e.g. an Avalanche photodiode (APD) photon counting detector.

(B) eFRET setup: The light source, preferably two LEDs, emits green and red light. The wide wavelength of the light emitted by the LEDs is preferably corrected with a filter, focused by an objective lens, and combined by a dichronic mirror, followed by the deflection of the light by a polychromic mirror into an objective lens and into a sample volume. Signals, e.g. fluorescence, originating from the molecule copies within the excitation volume, are collected by said objective lens, pass said polychromic filter and are split via a dichronic mirror into green and red fluorescence, which is then detected by a respective light detector, preferably a photomultiplier tube (PMT) or a silicon photomultiplier (siPM).

(C) ES-histogram depicting efficiency ($E$; $x$-axis) vs stoichiometry ($S$; $y$-axis) based on smFRET, wherein A-labelled, D-labelled, and A- and D- labelled copies can be distinguished along the diagonal of the plot.

(D) Signals obtained from an eFRET measurement. $F_{DD}$ summarizes signals from D-labelled, and A- and D- labelled molecule copies; $F_{AA}$ summarizes signals from A-labelled, and A- and D- labelled molecule copies; and $F_{DA}$ summarizes signals from A- and D- labelled molecule copies as well as noise signals, e.g. due to leakage and/or direct excitation.

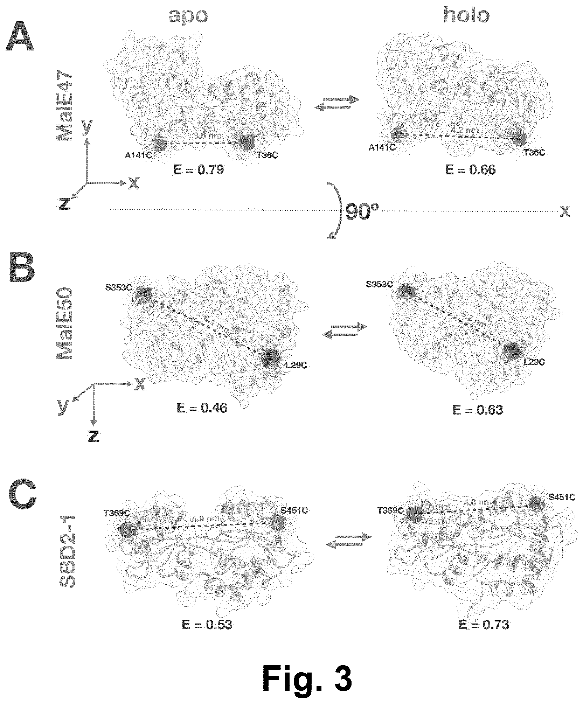

**Figure 3:** Crystal structures of exemplarily investigated proteins are shown in open (apo) and closed (holo) conformation, wherein dots denote the fluorophore pair positions. Distances between donor and acceptor fluorophores (dashed lines) are measured from the carbons of the peptide backbone. $E$ stands for the single molecule accurate FRET efficiency at the respective conformation. Yellow depicts a ligand of the respective protein.

(A) The protein MalE47 has two mutations in its backbone (A141C, and T36C), resulting in a fluorophore pair distance of 3.6 nm in its open conformation (PDB ID: 1lls) and of 4.2 nm in its closed conformation (PDB ID: 1anf).

(B) The protein MalE50 has two mutations near its binding pocket (S353C, and T29C), resulting in a fluorophore pair distance of 6.1 nm in its open conformation and of 5.2 nm in its closed conformation.

(C) The protein SBD2-1 has two mutations near its binding pocket (T369C, and S451), resulting in a fluorophore pair distance of 4.9 nm in its open conformation (PDB ID: 4kr5) and of 4.0 nm in its closed conformation. (PDB ID: 4kqp).

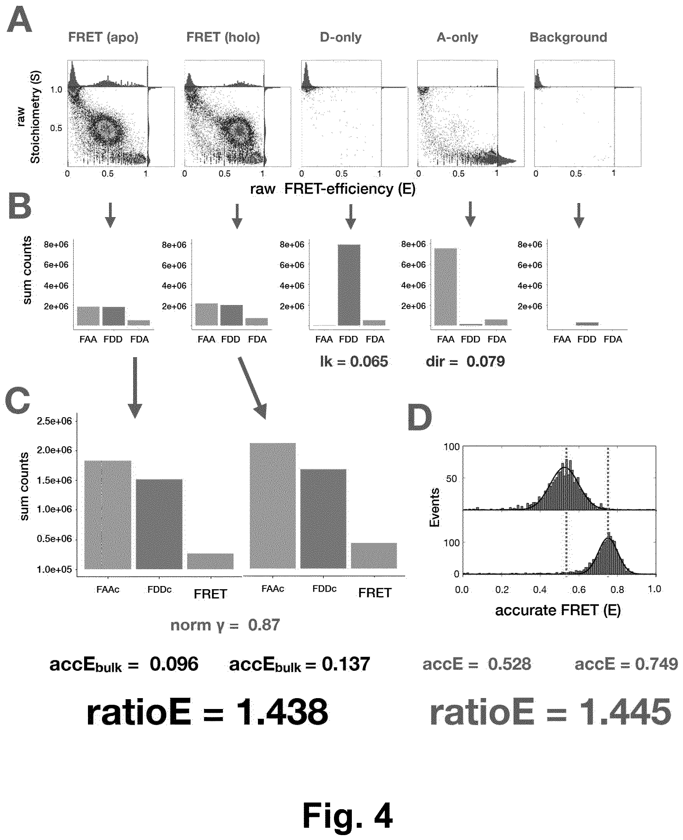

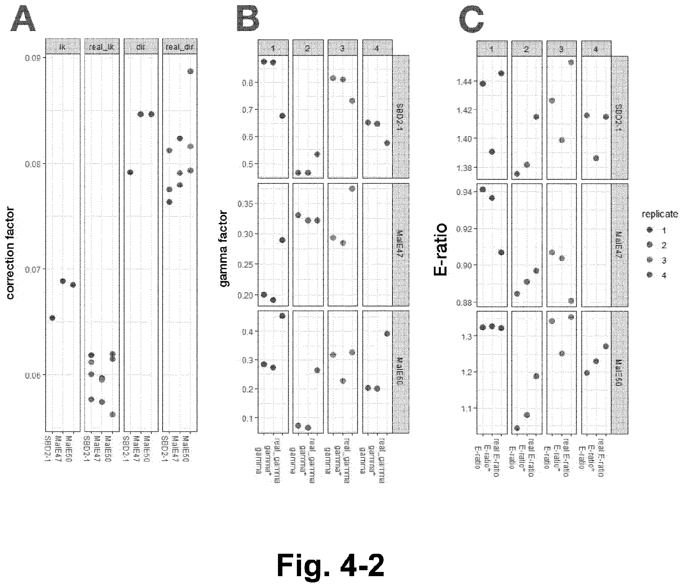

**Figure 4:** SBD2-1 smFRET data conversion.

(A) Raw ES-histograms in the proteins open (apo) and closed (holo) conformation. Furthermore ES-histograms are depicted for D-labelled, and A-labelled proteins copies, respectively as well as background, wherein imaging buffer was measured in the absence of the protein.

(B) Converted data from smFRET to eFRET analogue.

(C) Apo (left) and Holo (right) converted data corrected for noise signals, e.g. due to differences in background brightness, leakage and/or direct excitation. After said normalization, which resulted in $F_{AAcor}$ a $\gamma$ correction factor of 0.87 was obtained. The resulting Accurate eFRET efficiencies $E_{real}$ ($accE$) were 0.096 and 0.137 for the apo and holo conformation, respectively. The obtained $accE$ ratio was 1.438.

(D) Accurate smFRET efficiencies ($accE_{sm}$) of the apo ($accE_{sm}$ = 0.528) and holo ($accE_{sm}$ = 0.749) measurements depicted in (A) after smFRET data processing. The obtained $accE_{sm}$ ratio was 1.445.

**Fig. 4-2:** Output Comparison of eFRET and smFRET data processing.

(A) Crosstalk correction factors. lk and real_lk denote the leakage coefficient obtained from eFRET and smFRET, respectively; dir and real_dir the direct excitation coefficient obtained from eFRET and smFRET, respectively.

(B) Gamma correction factors per replicate and model protein. gamma denotes the gamma correction factor obtained from eFRET, gamma* the gamma correction factor obtained from eFRET corrected with smFRET correction factors (real_lk and real_dir, cf. (A)), and real_gamma denotes the gamma correction factor obtained from smFRET.

(C) E-ratios per replicate and model protein. E-ratio denotes the E ratio obtained from eFRET, E-ratio* the E ratio obtained from eFRET corrected with smFRET correction factors (real_lk, real_dir, cf (A); real_gamma, cf. (B)); and real E-ratio denotes the E ratio obtained from smFRET.

**Figure 5:** Boxplots of raw data are depicted per protein and signal type. Apo conformations are denoted with a dot; holo conformations are depicted with a triangle. Raw eFRET efficiencies (*raw $E_{bulk}$*) were obtained using the $F_{DD}$ and $F_{DA}$. The resulting Z-factors were 0.97 for SBD2-1 (left panel), 0.925 for MalE50 (middle panel) and 0.89 for MalE47 (right panel).

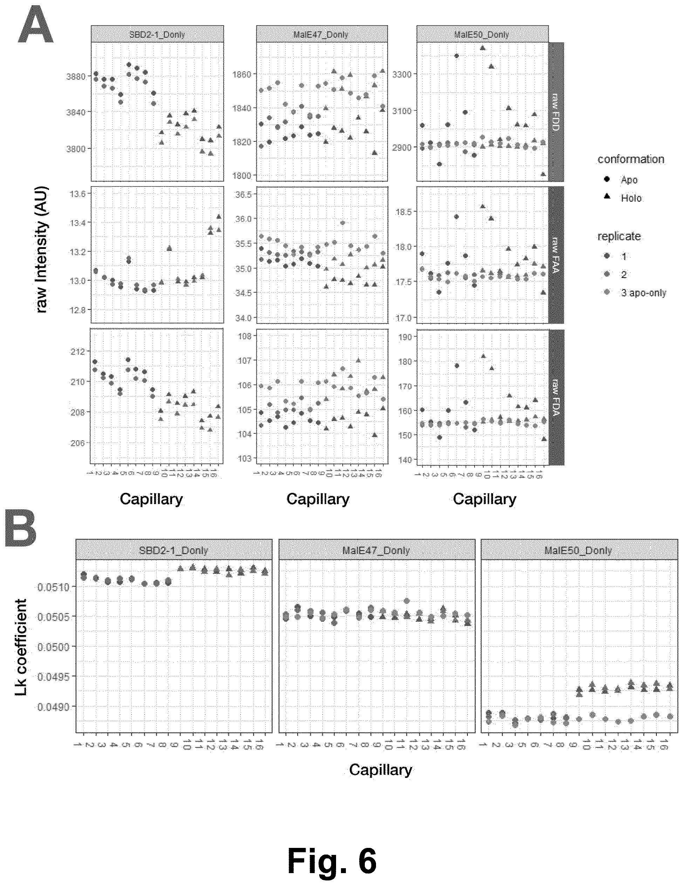

**Figure 6:** Leakage coefficient $c_{lk}$.

(A) Measurements of D-only labelled molecule copies depicted per protein and signal type. Apo conformations are denoted with a dot; holo conformations are depicted with a triangle. The 3rd replicate was measured only in its open conformation.

(B) $c_{lk}$ (*Lk coefficient*) for each capillary.

**Figure 7:** Direct excitation coefficient $c_{dirE}$.

(A) Measurements of A-only labelled molecule copies depicted per protein and signal type. Apo conformations are denoted with a dot; holo conformations are depicted with a triangle. The 3rd replicate was measured only in its open conformation.

(B) $c_{dirE}$ (*Dir coefficient*) for each capillary.

**Figure 8:** Normalization of corrected $F_{DD}$ and $F_{DA}$.

(A) Boxplot of corrected $F_{DD}$ before and after normalization ($F_{DDcor}$ and *norm $F_{DD}$*) per protein.

(B) Boxplot of $F_{DA}$ (*FRET*) and normalized $F_{DA}$ (*norm FRET*) per protein.

**Figure 9:** Data processing and comparison between normalized and non-normalized data for the determination of accurate E ratios (*accurate FRET (E))*.

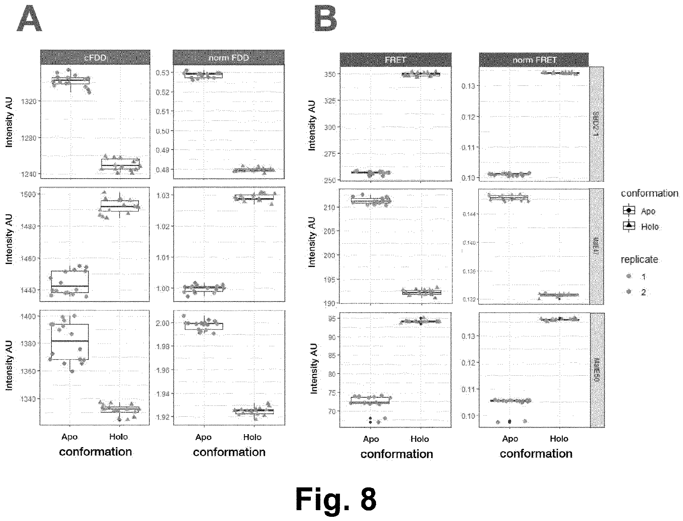

(A) $\gamma$-correction factors obtained from data shown in **Figure 8.**

(B) Accurate eFRET efficiencies *($E_{real}$)* obtained with the non-normalized and normalized $\gamma$-correction factors shown in (A).

(C) $E_{real}$-ratios obtained from normalized and non-normalized $E_{real}$ shown in (B).

(D) smFRET measurements of apo and holo conformation per protein and respective accurate FRET efficiencies. The resulting FRET ratios (*real Ratio*) were 1.43 for SBD2-1, 0.87 for MalE47, and 1.36 for MalE50.

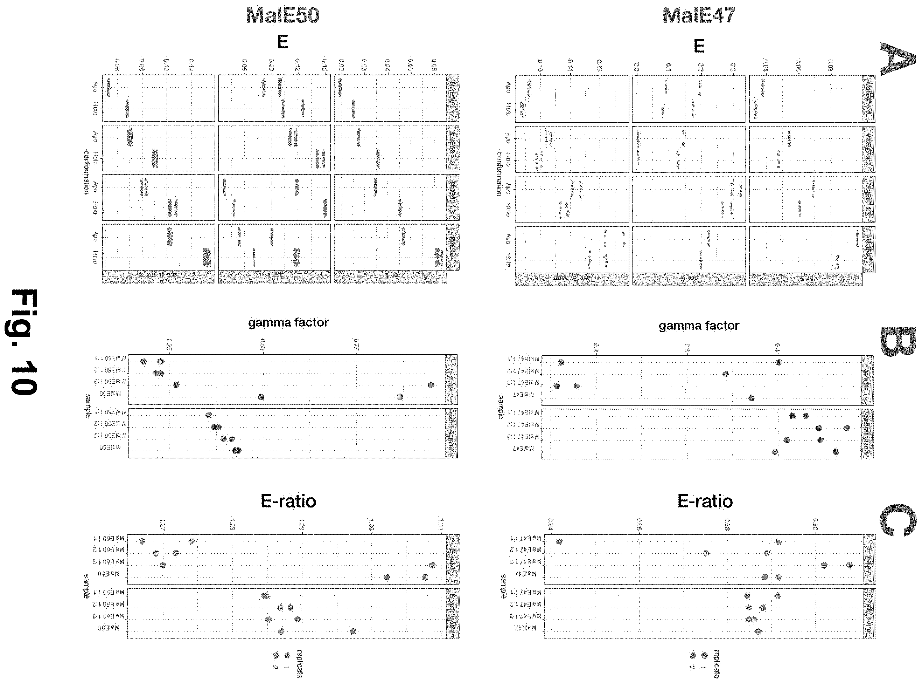

**Figure 10:** Donor only titration & resulting accE-ratios.

(A) eFRET efficiencies of MalE47 and MalE50 per titration step, wherein *1:1* refers to a one to one mixture of D-only labelled and D- and A-labelled molecule copies; *1:2* refers to a one to two mixture of D-only labelled and D- and A-labelled molecule copies; *1:3* refers to a one to three mixture of D-only labelled and D- and A-labelled molecule copies. *pr_E* refers to proximity FRET efficiency corrected for leakage and/or direct excitation. *acc_E* refers to $E_{real}$, wherein additionally to the correction as in case of *pr_E* the non-normalized $\gamma$-correction factor depicted in (B) was applied. *ace_norm* refers to $E_{real}$, wherein additionally to the correction as in case of *pr_E* the normalized $\gamma$-correction factor depicted in (B) was applied. (B) Comparison of normalized and non-normalized $\gamma$-correction factors per protein and titration step. (C) Comparison of normalized and non-normalized $E_{real}$-ratios per protein and titration step.

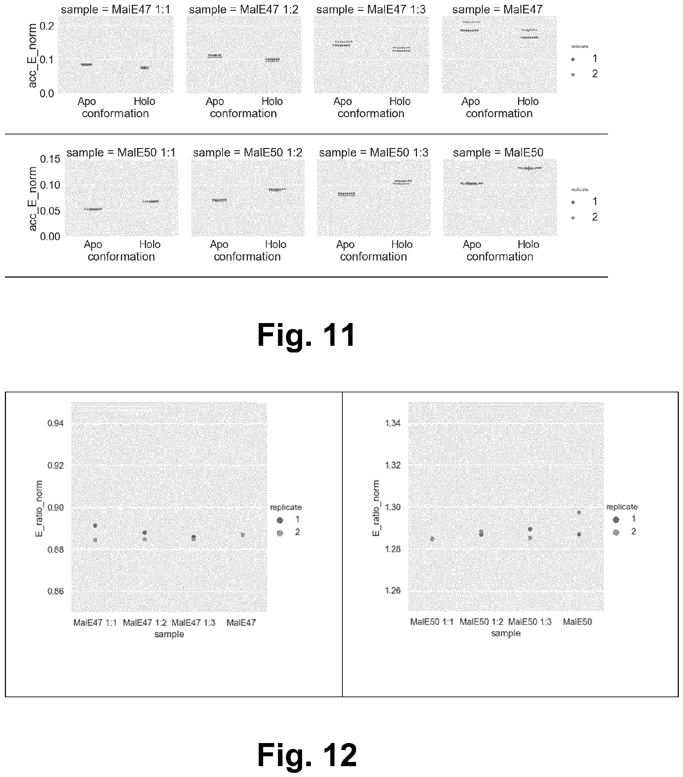

**Figure 11:** The corrected energy transfer efficiencies which are influenced by the amount of donor-only labeled protein for MalE47 (upper panel) and MalE50 (lower panel).

**Figure 12:** The energy efficiency ratios, which are independent of the amount of donor-only labeled molecules present for MalE47 (left panel) and MalE50 (right panel).

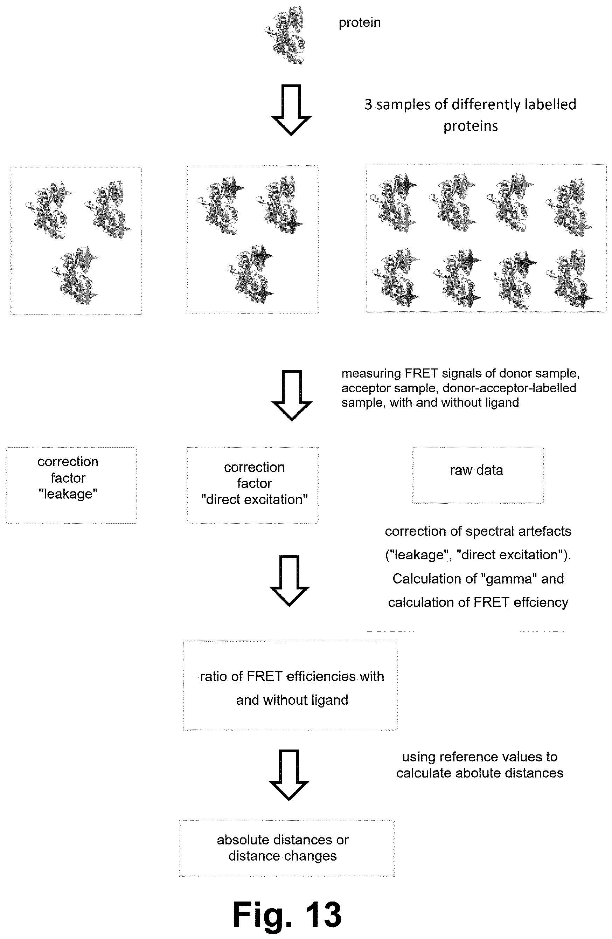

**Figure 13:** A schematic flow chart illustrating methods steps of the present invention with an exemplary labelled protein.

Detailed description

In the following, general principles of the present invention will be discussed in further detail and discussed based on illustrative examples or preferred embodiments of the present invention.

In particular, the present invention relates in a first aspect to a method for obtaining quantitative information on average donor-acceptor distance changes within a molecule, between molecules, within a molecule complex or between molecule complexes using ensemble Förster resonance energy transfer (eFRET), In the following reference is made to molecule(s) which is understood to cover any kind of molecules, e.g., biomolecules or molecule complexes. The method comprising the steps of: obtaining at least a donor sample, wherein said donor sample comprises at least copies of a free donor and/or donor-labelled copies of said molecule. It is preferred to use either a sample with free donor, i.e., donor which is not bound to a molecule or a purified donor-labelled sample, i.e. a sample in which remaining free donor is removed. However, since a sample can not 100% purified, also donor samples which comprises free donor and donor-labelled copies can be used. The same is true for an acceptor sample, i.e., the acceptor sample comprises at least copies of a free acceptor and/or acceptor-labelled copies of said molecule. Moreover, a donor-acceptor-labelled sample is obtained which comprises donor-acceptor-labelled copies of said molecule. It is preferred to perform an eFRET measurement for each of said samples under a first and a second condition to obtain for each of the samples a first eFRET result under the first condition and a second eFRET result under the second condition, wherein each eFRET measurement is performed using multiple respective molecule copies. In summary, this results in six eFRET measurements. In case the donor sample comprises only free donor, it may be also sufficient to perform only one eFRET measurement for said sample, either under the first or second condition. Also in case the acceptor sample comprises only free acceptor, it may be sufficient to perform only one eFRET measurement for said sample, either under the first or second condition. Thus, only four eFRET measurements may be performed, since one donor sample and one acceptor sample eFRET measurement may be omitted. It would be also possible to omit only one of the donor sample and acceptor sample eFRET measurements which would result in five eFRET measurements. Next, the first and second eFRET results are corrected for fluorophore-specific, condition-specific and/or inter-condition effects. For instance, determining condition-specific eFRET efficiencies are preferably based on the respective corrected first and second eFRET result. Quantitative information on average donor-acceptor distance changes can be determined within the molecule under the first and the second condition using such condition-specific eFRET efficiencies.

As mentioned above, the term molecule as used in the present application is not limited to a specific kind of molecule, e.g., a molecule can be a nucleic acid, a protein, an organic polymer or a complex thereof. Furthermore, said molecule can be a naturally occurring, biochemically and/or synthetically modified molecule, a synthetic molecule, or a combination thereof. Herein, the term "nucleic acid" comprises DNA and RNA as well as PNA and LNA and other non-natural "nucleic acids", wherein DNA refers to deoxyribonucleic acid and RNA to ribonucleic acid. Both DNA and RNA are built up of nucleotides consisting of a nitrogenous base, a five-carbon sugar, and at least one phosphate group. The term "DNA" encompasses for example genomic DNA and cDNA, and the term "RNA" comprises for example, mRNA, tRNA, miRNA, lncRNA, rRNA, asRNA, hnRNA, siRNA, snoRNA, snRNA, piRNA, and ribozymes. Herein, the term "protein" encompasses any kind of amino acid sequence. An amino acid sequence refers to a chain of amino acids, which are linked via peptide bonds, and is at least 5 amino acids long, more preferably at least 10 amino acids, even more preferably at least 50, 100, 200 or 500 amino acids. Thus, the term "protein" covers short peptides such as oligopeptides, polypeptides, proteins, protein fragments, i.e. parts of proteins such as biologically active or antigenic parts including epitopes, as well as protein complexes including oligoproteins and/or protein aggregates. As regards the function of the protein, there is no limitation.

For obtaining quantitative information on conformational changes of the molecule under study using the method according to the present invention, several copies of the molecule are obtained. If for example the molecule under study is a given protein A, several copies thereof (thus, an ensemble), i.e. several proteins A, are used in the method according to the invention. Said copies of the molecule are preferably completely or almost completely identical, wherein almost completely identical refers to minor differences in the copies for example due to biological variation, e.g. mutations. Thus, two copies of the molecule under study may differ in up to 10 positions, preferably in less than 5 positions, more preferably in less than 2 positions. Herein, the term "position" refers to a base or a base pair in case the molecule under study is a nucleic acid and/or to amino acids in case the molecule under study is a protein. Alternatively, two copies of the molecule under study may differ in up to 5% of the positions, preferably in less than 2.5% of the positions, more preferably in less than 1% of the positions. Differences in positions between molecule copies can be determined, e.g., by sequence alignments.

Preferably, the molecule under study is obtained in a fluid, preferably a liquid such as a solution. By choosing the type and/or composition of the fluid according to the characteristics of the respective molecule under study, for example the molecule's function and/or activity can be maintained during analysis and/or undesired degradation and/or conformational changes avoided. Thus, the solution may comprise for example a buffer. The buffer may be chosen individually for each molecule under study and in view of the experimental conditions to be tested. Preferably, the buffer is free of quenching molecules, reduces the occurrence of the molecule copies being attached to the measurement vessel, and/or reduces the risk of aggregation, e.g. of molecule copies. The present invention, however, is not limited to "free" molecules in a liquid. As discussed below, molecules may be provided at an outer surface of a cell, within a cell, within the membrane of a cell, on the surface of a microsphere or on the surface of the measuring vessel, e.g. microwell bottom or capillary wall. For example, the cell can be an eukaryotic cell or a prokaryotic cell. Molecules may also be provided at an outer surface of a virus or virus-like-particle, within a virus or virus-like-particle.

According to the method of the invention a sample is obtained, wherein the sample comprises multiple copies of the molecule, preferably in a solution. Such a sample has preferably a volume between $0.1\mu l$ and $200\mu l$, more preferably between $1\mu l$ and $50\mu l$ and/or comprises said multiple copies of the molecule in a concentration between 10 pM to $10\ \mu M$, preferably in the range of 1 nM to $1\ \mu M$, and more preferably about 50 nM. In case the molecule concentration is higher, the sample comprising the molecule copies is preferably diluted to said concentration using, e.g., a buffer. More specifically, at least one, preferably three samples are obtained, each comprising multiple molecule copies that are sample-specific labeled with a donor, an acceptor, or both.

The donor is preferably a fluorophore, e.g. a first fluorophore (which is also called fluorescent dye or simply dye in the present application). A fluorophore refers to a fluorescent compound that can (re-)emit light upon light excitation. Fluorophores suitable for (e)FRET can have a spectral range from ultraviolet to infrared (IR), e.g. from about 250 nm to 850 nm. Due to their high extinction coefficients, red and near-infrared fluorophores often emit comparatively strong but have consequently often comparatively short fluorescence lifetime (< 4 ns). Furthermore, fluorescent amino acids, i.e. tyrosine, tryptophan, cysteine, and/or lysine, which are endogenously present in (oligo-)peptides and/or proteins, can be used as fluorophores.

The acceptor is preferably a fluorophore (fluorescent dye, dye), e.g. a second fluorophore. As regards the fluorophore, the same applies as described above in case of the donor being a fluorophore. Hence, it is preferred that the donor is a fluorophore and the acceptor is a fluorophore.

If both donor and acceptor are a fluorophore, it is preferred that the donor, e.g. the first fluorophore, and the acceptor, e.g. the second fluorophore, are different fluorophores. This has the advantage that a FRET signal can be obtained due to the occurrence of donor-stimulated fluorescence of the acceptor. Furthermore, a single photoconvertible fluorophore may be used, e.g. a fluorophore that behaves comparable to the Eos fluorescent protein from Lobophyllia hemprichii. Thus, the fluorophore's fluorescence could switch from green (about 516 nm) to red (about 581 nm) upon UV irradiation (about 390 nm), wherein the green and red variants of Eos can generate FRET

It is furthermore preferred that the donor has an emission spectrum and the acceptor an excitation spectrum, and that the emission spectrum of the donor overlaps with the excitation spectrum of the acceptor. This is advantageous as the overlap of the spectra is required for the occurrence of FRET. In other words, this is advantageous as the donor non-radiatively transfers energy to the acceptor.

More specifically, it is preferred that the donor has a fluorescence emission spectrum and the acceptor an excitation spectrum, and that the fluorescence emission spectrum of the donor overlaps with the excitation spectrum of the acceptor. This is advantageous as a fluorescence emission of the donor can excite an emission of the acceptor.

In case donor and acceptor are fluorophores, preferred fluorophore pairs include, e.g., Alexa 546 and Alexa 647 dyes, other Alexa and Atto dyes, cyan fluorescent protein (CFP) and yellow fluorescent protein (YFP), green fluorescent protein (GFP) and rhodamine and derivatives, fluorescein and derivatives and cyanine dyes such as Cy3, cy5, cy5.5, cy7 and derivatives thereof, as well as europium and allophycocyanin.

The method according to the invention preferably comprises the step of obtaining at least a donor sample which preferably comprises donor-labelled copies of said molecule, an acceptor sample which preferably comprises acceptor-labelled copies of said molecule, and a donor-acceptor-labelled sample comprising donor-acceptor-labelled copies of said molecule. Instead of providing donor-labelled copies and acceptor-labelled copies of the molecule, it is also possible to use donor and/or acceptor samples which comprise the (pure) fluorophore in a "free" state. Moreover, in case intra-molecular distances should be measured, the donor-acceptor-labelled copies are provided of copies of one molecule, i.e., copies of the same molecule are labelled with the donor and the acceptor. In case of inter-molecular distance measurements, it is preferred to label copies of a first molecule with the donor and copies of a second molecule with the acceptor.

In particular, at least three samples are preferably obtained, i.e. at least a first, a second, and a third sample, wherein at least the third sample comprises multiple copies of the molecule(s). The first and second sample may contain only donor fluorescent dye and acceptor fluorescent dye, respectively. According to a preferred embodiment, the first sample comprises molecule copies which are labelled with the donor, and the second sample comprises the molecule copies which are labelled with the acceptor. The molecule(s) copies comprised in the third sample are preferably labelled with both the donor and the acceptor. Thus, at least three samples are preferably obtained using a single donor-acceptor pair, wherein said at least three samples preferably comprise the molecule under study either labelled with i) the donor at a specific donor-labelling site within the molecule, ii) the acceptor at a specific acceptor-labelling site within the molecule, or iii) both, respectively. According to a further preferred embodiment, the molecule comprises at least two labelling sides which are not specific for the donor and the acceptor, i.e., the donor may label any one of the at least two labelling sites and the acceptor may also label any of the at last two labelling sites. Thus, the labelling of the at least two-labelling sites is performed stochastically by the donor and the acceptor.

Optionally, further samples are obtained. This might be advantageous for obtaining quantitative information on average donor-acceptor distance changes within a molecule using eFRET and i) more than one donor-acceptor pair and/or ii) a single donor-acceptor pair with more than one donor- or acceptor-labelling site within the molecule, and/or iii) a combination thereof.

Labeling can be done by using appropriate (bio-)chemistry. For example, the molecule under study can be a protein that may be his-tagged. Then, an Ni-NTA-based dye can be used to label the protein at the his-tag. Alternatively, the respective protein copies can be stochastically labelled, e.g., with Alexa 546 and Alexa 647 dyes using maleimide chemistry. Other labeling strategies include the use of unnatural amino acids and biorthogonal labeling methods such as Click-chemistry or labelling via tags, encoded in the protein structure, such as the aforementioned his-tag, but also including tags like snap-tag or avi-tag. Alternatively, if a photoconvertible fluorophore is used, labelling can be performed in a single labelling step followed by UV irradiation for stochastic photoconversion of the fluorophore into acceptor and donor fluorophores, respectively.

Preferably, the same labeling batch is used for eFRET measurements, e.g. of the molecule being a protein in the presence or absence of a potential binding partner as first and second condition, respectively. Furthermore, upon labeling the labeled molecule copies are preferably purified before preforming an eFRET measurement, e.g. by size-exclusion chromatography. Thus, noise signals can be reduced in eFRET.

According to the inventive method, an eFRET measurement is performed for each of the at least three (labelled) samples, i.e. at least a donor sample, an acceptor sample, and a donor-acceptor labelled sample, under a first and a second condition.

Said first and second condition may differ from each other by the presence of a further molecule in one of the two conditions, wherein the further molecule preferably interacts with the molecule. Thus, it can be determined whether the conformation of the molecule under study is influenced and/or affected by the presence of the further molecule. This can occur in case the molecule under study and the further molecule are for example receptor and ligand, enzyme and substrate, enzyme and coenzyme, or enzyme and cofactor. This is for example especially advantageous in the context of drug screenings. Optionally or alternatively, also the concentration of the molecule under study or the further molecule can be varied between the first and the second condition and/or another further molecule added in one of the two conditions. This can be useful to determine binding affinities between the molecule and the further molecule and/or the impact of the presence of said another further molecule on the binding affinity between the molecule and the further molecule.

Optionally or alternatively, said first and second condition may differ from each other in at least one parameter selected from the group consisting of temperature, pH, salt content, buffer composition, the addition of chaotropes, the addition of excipients, ionic strength and the presence of binding partners. It is preferred that the first and the second condition differ from each other in only one parameter of said group. This is advantageous to assess whether the amended parameter influences the conformation of the molecule under study. For example, it can thus be determined at which temperature aggregation of the molecule under study begins and/or in case of the presence of a further molecule in both conditions, how a change in temperature affects a binding of the molecule and said further molecule.

Of note, an eFRET measurement can also be performed for each of the at least three labelled samples, i.e. at least a donor-, an acceptor-, and a donor-acceptor labelled sample, under more than only the first and the second condition. This is especially advantageous in case of screening approaches for example, wherein several potential interacting and/or binding partners of a molecule under study are to be investigated. In such an experimental setting, a first condition may refer to the molecule in the absence of any potential binding partner, a second condition to the presence of a first potential binding partner, a third condition to the presence of a second potential binding partner, a fourth condition to the presence of a third potential binding partner, a fifth condition to the presence of the second and the third potential binding partners, and so on. However, as regards the third, fourth, fifth, etc condition the same applies as it is disclosed herein with respect to the (first or) second condition.

As according to the inventive method, an eFRET measurement is performed for each of the at least three (labelled) fluorogenic samples and at least under a first and a second condition for the donor-acceptor-labelled sample, in total preferably six eFRET, but at least four, measurements are performed.

For eFRET, the donor is to be excited directly and the acceptor is to be excited either directly or indirectly via the donor. Therefore, light having a wavelength within the excitation spectrum of the donor is preferably transmitted to the respective sample to excite the donor. Furthermore, also light having a wavelength within the excitation spectrum of the acceptor is preferably transmitted to the respective sample to excite the acceptor.

It is particularly preferred that light is transmitted to the respective sample from a monochromatic light source, preferably from an LED, to excite the donor and/or the acceptor. An LED is especially preferred as it has the advantage of being much cheaper than other sources of monochromatic light such as a laser.

It is furthermore preferred that, if the acceptor is a fluorophore, the emission of the acceptor is measured, *preferably the fluorescence emission of the acceptor.* Hence, in case donor and acceptor are in close proximity to each other, the excited donor can excite the acceptor by non-radiative energy transfer and an eFRET signal may be observed, i.e. upon excitation of the donor an emission of the acceptor.

Furthermore, it is preferred to transmit light having a wavelength within the excitation spectrum of the donor and light having a wavelength within the excitation spectrum of the acceptor in an alternating manner to the respective sample for an eFRET measurement. In particular, an alternating scheme is preferably used. According to the present invention, the two wavelengths of the light sources may be emitted simultaneously or subsequently. This has the advantage that correction factors for determining accurate molecular distances and/or for obtaining quantitative information on average donor-acceptor distances within a molecule can be easily obtained.

Hence, the method according to the present invention preferably comprises a step of transmitting light to the respective sample in at least one illumination cycle, preferably 1-500 illumination cycles, wherein an illumination cycle has preferably 2 light pulses between 10 ms and 1s sec, preferably light pulses with light having alternately a wavelength within the excitation spectrum of the donor and the acceptor, respectively.

For example, a sample can be illuminated according to the following illumination cycle: illuminating the sample with excitation light of the lower (shorter) wavelength range to excite the donor, during said illumination with donor excitation light: recording the light emitted at donor and acceptor emission wavelength ranges (the signals being referred to herein as $"F_{DD}"$ and $"F_{DA}"$, respectively), switching off the illumination, illuminating the sample with excitation light of the higher (longer) wavelength range to excite the acceptor, during said illumination with acceptor excitation light: recording the light emitted at the higher (longer) emission wavelength range (acceptor emission, herein referred to as $"F_{AA}"),$ switching off the illumination. Said cycle can be repeated several times, preferably 2-100 times. Of note, the order of the two illumination steps - and the respective subsequent recording step - can be switched within any illumination cycle.

Thus, it is preferred that light from an LED is transmitted to multiple labelled molecule copies comprised in a given sample in at least one illumination cycle.

As an eFRET measurement is preferably performed using at least one illumination cycle, but preferably using a plurality of illumination cycles, the measured emission of the acceptor and the donor, respectively, is preferably averaged over said illumination cycles.

According to the inventive method, an eFRET measurement for each of said samples is preferably performed under a first and a second condition to obtain for each of the samples a first eFRET result under the first condition and a second eFRET result under the second condition, wherein each eFRET measurement is performed using multiple respective molecule copies.

As an eFRET measurement is based on the detection of emission, preferably fluorescence emission, of a respective sample in two emission wavelength ranges, the first and the second eFRET results preferably each comprise respective measurement values of the emission of the acceptor after donor excitation ($F_{DA}$), the emission of the acceptor after acceptor excitation ($F_{AA}$), and/or the emission of the donor after donor excitation ($F_{DD}$).

It is particularly preferred that respective measurement values are obtained of $F_{DA}$ for the donor sample, the acceptor sample, and the donor-acceptor-labelled sample, of $F_{AA}$ for the acceptor sample and the donor-acceptor-labelled sample, and of $F_{DD}$ for the donor sample and the donor-acceptor-labelled sample. Thus, a condition-specific eFRET result preferably comprises seven emission measurement values which is advantageous to reduce the required measurement time per condition.

*Background correction*

Furthermore, it is preferred that the obtained first and second eFRET results are corrected for background signals. Therefore, a background measurement is preferably performed using a sample not comprising any molecule copy under study. Preferably, said background measurement is performed using a further sample that corresponds to the samples under study, e.g., at least the donor sample or donor-labelled sample, the acceptor sample or acceptor-labelled sample, and the donor-acceptor labelled sample, except the respective labelled molecule copies. Thus, preferably the same components other than the molecule under study, such as buffer, salts etc., are comprised in said further sample as in the samples under study. preferably even in the same concentrations. In case the first and the second condition differ from each other by the presence of a further molecule such as a potential binding partner of the molecule under study, the further sample used for the background measurement preferably further comprises said further molecule, preferably in the same concentration as at least one of the samples under study. Thus, sensitivity and accuracy of the inventive method can be increased.

As such a background measurement is independent from the molecule under study, it may only be performed once for a given sample composition (i.e. a given buffer, pH, etc as the samples under study but without the respective molecule of interest). Thus, the results obtained from such a background measurement are preferably stored, e.g. in a database, from which they can be obtained if required. This is advantageous as it avoids repeated background measurements of a given sample composition in the absence of the molecule under study and can thus accelerate, e.g. a high throughput screening for potentially interacting molecules.

According to the inventive method, the obtained first and second eFRET results are corrected, preferably for fluorophore-specific, condition-specific and/or inter-condition effects. This is advantageous to further increase accuracy and sensitivity of the inventive method.

More specifically, it is preferred that the step of correcting the obtained first and second eFRET results for fluorophore-specific, preferably condition-specific and/or inter-condition effects comprises the steps of determining fluorophore- and/or condition-specific correction factors using the respective first and second eFRET result of donor sample and acceptor sample, correcting the first and second eFRET results of the donor-acceptor-labelled sample using the respective fluorophore- and/or condition-specific correction factors, as well as of determining an inter-condition correction factor using the condition-specific corrected first and second eFRET results of the donor-acceptor-labelled sample, and correcting the corrected first and second eFRET results of the donor-acceptor-labelled sample using the inter-condition correction factor.

In particular, the method of the present invention may be based on fluorophore-specific correction factors. In this case, the respective first and second eFRET result preferably comprises the steps of determining a leakage coefficient $c_{lk}$, and/or of determining a direct excitation coefficient $c_{dirE}$. This is advantageous to correct for crosstalk between the detection channels before determining eFRET efficiencies. The method of the present invention may also consider condition-specific correction factors, preferably in cases where the donor sample is measured under two different conditions and/or the acceptor sample is measured under two different conditions. Hence, the the step of determining condition-specific correction factors using the respective first and second eFRET result preferably comprises the steps of determining a condition-specific leakage coefficient $c_{lk}$, and/or of determining a condition-specific direct excitation coefficient $c_{dirE}$. This is advantageous to correct for crosstalk between the detection channels before determining condition-specific eFRET efficiencies. The condition-specific coefficients are typically also fluorophore-specific.

Leakage and/or direct excitation coefficients can be determined based on single-labelled samples, e.g., preferably donor-labelled and acceptor-labelled samples, but also based on the donor fluorophore and the acceptor fluorophore which are not labelled. For example, using donor sample excited at donor excitation wavelengths, the leakage of donor emission into the acceptor channel(s) can be assessed. Furthermore, with an acceptor sample excited using the donor-excitation wavelength and using the acceptor excitation wavelength, the direct excitation of acceptor may be assessed.

More specifically, the leakage coefficient $c_{lk}$ is determined based on the ratio of $F_{DA}$ and $F_{DD}$, wherein said $F_{DA}$ and $F_{DD}$ are obtained for the donor sample, preferably for the donor-labelled sample or the donor fluorophore, preferably under the respective condition(s). Determining $c_{lk}$ as disclosed herein is advantageous as it describes the emission of the donor that leaks through the acceptor detection channel(s) and/or filter(s) and thus, is detected as acceptor emission. In particular, the leakage coefficient $c_{lk}$ is preferably determined based on the donor sample according to formula (II):

$$c_{lk} = \frac{F_{DA}}{F_{DD}} \qquad \text{(II)},$$

wherein $F_{DA}$ denotes the respective acceptor emission after donor excitation, and $F_{DD}$ the respective donor emission after donor excitation.

Optionally or alternatively, the direct excitation coefficient $c_{dirE}$ is preferably determined based on the ratio of $F_{DA}$ and $F_{AA}$, wherein said $F_{DA}$ and $F_{AA}$ are obtained for the acceptor sample, preferably under the respective condition(s). Determining $c_{dirE}$ as disclosed herein is advantageous as it describes the emission of the acceptor after direct excitation by the donors' excitation light and thus, without any FRET taking place. In particular, the $c_{dirE}$ is preferably determined based on the acceptor sample, preferably on the acceptor-labelled sample or based on the free acceptor fluorophore, according to formula (III):

$$c_{dirE} = \frac{F_{DA}}{F_{AA}} \qquad \text{(III)},$$

wherein $F_{DA}$ denotes the respective acceptor emission after donor excitation, and $F_{AA}$ the respective acceptor emission after acceptor excitation.

Preferably, the step of correcting the first and second eFRET results using the respective correction factors comprises the step of determining a corrected $F_{DA}$ ($F_{DAcor}$ or $F_{DA\ corrected}$) for the donor-acceptor-labelled sample by subtracting from $F_{DA}$ the $c_{lk}$ corrected $F_{DD}$ and the $c_{dirE}$ corrected $F_{AA}$, wherein said $F_{DA}$, $F_{DD}$, and $F_{AA}$ are measured for the donor-acceptor-labelled sample under the respective condition. Thus, a corrected $F_{DA}$ ($F_{DAcor}$) for the donor-acceptor-labelled sample is preferably obtained according to the following formula:

$$F_{DAcor} = F_{DA} - c_{lk} \times F_{DD} - c_{dirE} \times F_{AA} \qquad (IV).$$

The step of determining the inter-condition correction factor using the corrected first and second eFRET results comprises the step of determining a $\gamma$-correction factor based on the negative ratio of i) the difference of $F_{DAcor}$ between the first and the second condition and ii) the difference of $F_{DD}$ of the donor-acceptor-labelled sample between the first and the second condition.

More specifically, the $\gamma$-correction factor is calculated using the double-labelled sample, i.e. the donor-acceptor-labelled sample, and calculating the following ratio:

$$\gamma = - \frac{\Delta F_{DA}}{\Delta F_{DD}} \qquad (V),$$

wherein

$$\Delta F_{DA} = F_{DA}{}^{condition\ 1} - F_{DA}{}^{condition\ 2} \qquad (VI),$$

and wherein

$$\Delta F_{DD} = F_{DD}{}^{condition\ 1} - F_{DD}{}^{condition\ 2} \qquad (VII).$$

Of note, for calculating the $\gamma$-correction factor no further eFRET measurement is required, only an additional calculation step.

Furthermore, the obtained data are preferably normalized using the $F_{AA}$ to mitigate effects of different molecule copy concentrations in the respective samples due to pipetting errors. Thus the signals $F_{xx}{}^{condition\ y}$ are replaced by $F_{xx}{}^{condition\ y} / F_{AA}{}^{condition\ y}$ in the formulas above.

Such that:

$$\Delta F_{DA} = F_{DA}{}^{condition\ 1}/F_{AA}{}^{condition\ 1} - F_{DA}{}^{condition\ 2}/F_{AA}{}^{condition\ 2} \qquad (VII),$$

and

$$\Delta F_{DD} = F_{DD}{}^{condition\ 1}/F_{AA}{}^{condition\ 1} - F_{DD}{}^{condition\ 2}/F_{AA}{}^{condition\ 2} \qquad (VII-2).$$

This is disadvantageous when the acceptor quantum yield changes significantly between the two conditions.

Alternatively, the acceptor can also be partially photobleached. An eFRET signal can then also be observed, i.e. a donor emission due to excitation by acceptor emission. In such an experimental setting, condition 1 and condition 2 refer to the situation before and after photobleaching, respectively. Such an experimental setting uses a photobleaching step and an additional measurement of $F_{DD}$ and $F_{DA}$ after photobleaching. The formulas above are then modified in that "condition 1" and "condition 2" are replaced by "before photobleaching" and "after photobleaching" respectively. However, no further sample needs to be prepared. Moreover, such an experimental setting has the advantage that differences in molecule copy concentrations in the samples must not be corrected. Furthermore, calculating the $\gamma$-correction factor using photobleaching has the advantage that the $\gamma$-correction factor can be calculated for different conditions separately. While for the $\gamma$-correction using two different conditions, one assumes a constant $\gamma$, using photobleaching samples of all conditions can be subjected to acceptor photobleaching and thus for all conditions a separate $\gamma$ can be determined.

According to the inventive method, condition-specific eFRET efficiencies are determined based on the respective corrected first and second eFRET result.

Therefore, condition-specific $\gamma$-corrected $F_{DD}$ ($F_{DDcor}$) for the donor-acceptor-labelled sample under the respective condition are preferably determined as follows:

$$F_{DDcor} = \gamma * F_{DD} \qquad \text{(VIII)}.$$

Then, condition-specific eFRET efficiencies are preferably determined based on the ratio of i) the respective $F_{DAcor}$ and ii) the sum of the respective $F_{DAcor}$ and the respective $\gamma$-corrected $F_{DD}$. More specifically, a condition-specific eFRET efficiency is calculated according to the following formula (IX):

$$E_{app} = \frac{F_{DAcor}}{F_{DAcor} + F_{DDcor}} \qquad \text{(IX)},$$

wherein $E_{app}$ refers to the apparent, i.e. measured, eFRET efficiency of the molecule under study under a given condition, which includes the aforementioned conditions (e.g. ligand presence, pH, etc.), but also the labeling batch of donor-acceptor-labelled molecule.

The apparent eFRET efficiency and the accurate eFRET efficiency ($E_{real}$) of the energy transfer are related to each other as follows:

$$E_{app} = E_{real} \frac{c_{DA}}{c_{DA} + c_D} \qquad \text{(X)},$$

wherein $c_{DA}$ refers to the concentration of the double-labeled molecule copies, i.e. the acceptor-donor-labelled molecule copies, and wherein $c_D$ refers to the concentration of the donor-labeled molecule copies in the respective sample. In this case, the accurate eFRET efficiency $E_{real}$ depends only on the condition (e.g. ligand presence, pH, etc.), but not on the labeling batch of donor-acceptor-labelled molecule and is a physical property of the molecule under study.

According to the inventive method, quantitative information on average donor-acceptor distance changes within the molecule or between two molecules under the first and the second condition is determined using the condition-specific eFRET efficiencies. Therefore, a ratio of obtained condition-specific apparent eFRET efficiencies is preferably calculated. This has the advantage that such a ratio-based approach is, largely or even completely, independent of labeling efficiencies and represents a quantitative measure, in case the same labeling batch of said molecule is used for obtaining the apparent FRET efficiency per condition.

More specifically, the accurate eFRET efficiency ($E_{real}$) of the energy transfer can be used to determine the distance between donor and acceptor within the molecule under study or between molecules under study. Therefore, information on a donor-acceptor specific Förster radius $R_0$ and on a donor-acceptor distance within the molecule under the first or the second condition r is preferably obtained.

The term "Förster radius" (i.e. $R_0$) refers to the distance at which 50% FRET occurs and is specific for a given donor-acceptor pair. Information on a Förster radius of a given donor-acceptor pair can be obtained, e.g. for many commonly used fluorophore pairs, from publicly available data sources such as databases and publications. It is usually calculated assuming random (inter-)fluorophore orientation.

Information on the distance of a respective donor-acceptor pair in at least one conformational state, e.g. of a receptor protein with and/or without ligand bound can be obtained by x-ray crystallography for example and is publicly available for many molecules.

Thus, information on a donor-acceptor pair specific Förster radius and on a donor-acceptor distance within the molecule under the first or the second condition r is preferably obtained, preferably from, e.g. publicly, available data sources such as databases and/or publications and/or from previous work. Using such information has the advantage that donor-acceptor distances within a molecule can also be determined for other conformational states using the eFRET efficiency ratio according to the inventive method. Hence, for example screening approaches for molecules that interact with the molecule under study, thus leading to a conformational change in the molecule under study, can be significantly accelerated, e.g. in case of drug screenings.

Based on the ratio of condition-specific $E_{app}$, the respective ratio of $E_{real}$ can then be determined as follows:

$$\frac{E_{app\ condition\ 1}}{E_{app\ condition\ 2}} = \frac{E_{real\ condition\ 1}}{E_{real\ condition\ 2}} \qquad\qquad (XI).$$

According to formula (I), $E_{real}$ is related to the donor-acceptor distance within a molecule under a given condition and the donor-acceptor specific Förster radius. Thus, quantitative information on the donor-acceptor distance within the molecule under study can be obtained under a further condition. Preferably, information on the donor-acceptor distance within the molecule under study under the first or second condition is obtained and therefore, using the following formulas (XII) and (XIII), information on the donor-acceptor distance under the respective other of the first and second condition can be obtained.

In the following, formulas (XII) and (XIII) are exemplarily shown for determining quantitative information on the donor-acceptor distance within the molecule under study under the second condition using information obtained on the donor-acceptor distance under the first condition, the donor-acceptor pair specific Förster radius, and the ratio of obtained condition-specific $E_{app}$:

$$\frac{E_{app\ condition\ 1}}{E_{app\ condition\ 2}} = \frac{r_2^6 + R_0^6}{r_1^6 + R_0^6} \qquad\qquad (XII),$$

and

$$r_2 = \sqrt[6]{\frac{E_{app\ condition\ 1}}{E_{app\ condition\ 2}} * (r_1^6 + R_0^6) - R_0^6} \qquad\qquad (XIII).$$

The $E_{real}$ can also be used to distinguish between different binding modes of ligands depending on the conformation change they elicit. An example would be a protein that undergoes conformational change when it processes a naturally occurring ligand such as ATP (e.g. open → closed with ATP bound → ATP hydrolosis and ejection → open). An inhibitor of such an enzyme can work by binding to the ATP binding pocket, leading to a constant close conformation (competitive inhibitor), or to a different part of the enzyme, leading to a constant open conformation, which doesn't allow stable binding of the natural ligand (allosteric inhibition). In the drug discovery workflow, knowledge of binding mechanism is very valuable information that can often only be reliably obtained by time-consuming, high-resolution structure methods that aren't amendable to high-throughput workflows (X-Ray, NMR, Cryo-EM) or error-prone competition assays that yield hard-to-interpret results. The present invention can provide a reliable method that is high-throughput ready without having a competing molecule present.

$E_{real}$ and the present invention in general can also be used to characterize inhibitors of protein-protein interactions as well as linkers creating de-novo protein-protein interactions (such as PROTACs). E.g. a PROTAC leading to closer binding of proteins would most likely lead to a higher $E_{real}$ than a PROTAC that only connects two proteins loosely.

The inventive method is preferably carried out by a computer.

The present invention relates in a further aspect to a computer program comprising instructions which, when the program is executed by a computer, cause the computer to carry out the method according to the first aspect of the invention.

The present invention relates in a further aspect to a computer-readable data carrier comprising instructions which, when executed by a computer, cause the computer to carry out the method according to the first aspect of the invention. Optionally or alternatively, the computer-readable data carrier comprises the computer program according to the second aspect of the invention.

Herein, the computer-readable data carrier is intended to be understood to encompass a computer-readable (long-time) storage medium such as an SSD, hard disk (HDD), USB memory, SD card, or any other memory which can be also located in a cloud, as well as a random-access memory for temporary storage.

The present invention also relates to a measurement system comprising a controller adapted for obtaining quantitative information on average donor-acceptor distance changes within a molecule using eFRET, *preferably in accordance with the method as discussed above.* For example, the measurement system may comprise a computer-readable data carrier according to the invention, preferably comprising the computer program according to an aspect of the invention the controller is adapted to execute.

The measurement system comprises further means for accommodating a vessel, preferably a microwell plate or a capillary, comprising a donor sample comprising donor-labelled copies of said molecule or free donor fluorophore, an acceptor sample comprising acceptor-labelled copies of said molecule or free acceptor fluorophore, and/or a donor-acceptor-labelled sample comprising donor-acceptor-labelled copies of said molecule; a monochromatic light source, preferably an LED, and a light detector, preferably a PMT or a silicon photomuliplier siPM; preferably a filter, a dichronic mirror, a polychronic mirror, and/or an objective lens; means for performing an eFRET measurement using multiple respective molecule copies; and control means adapted for controlling the means for accommodating the vessel; controlling the monochromatic light source for transmitting light from the monochromatic light source to the vessel; controlling the light detector for detecting signals from the vessel; and controlling said means for performing the eFRET measurement.

The present invention relates in a further aspect to the use of a measurement system, e.g., as discussed above, to obtain quantitative information on average donor-acceptor distance changes within a molecule using eFRET.

Other aspects and advantages of the present invention will be described in the following examples, which are given for purposes of illustration and not by way of limitation.

The present invention will be discussed more specifically with regard to two non-limiting examples, namely applying the method of the present invention in free solutions and applying the method of the present invention in samples where the labelled biomolecule is at the outer surface of a cell or even inside a cell.

In general, the measurement is preferably based on the detection of fluorescence of a fluorogenic sample at two excitation wavelength ranges and at two emission wavelength ranges. The measurement setup preferably comprises a sample chamber (e.g. a capillary or microtiter plate), preferably a light source which emits different wavelength ranges, wherein preferably two different light sources are used for the two different wavelength ranges. The measurement setup preferably also comprises several lenses, filters and/or dichroic mirrors to redirect, combine and separate light as well as at least one detector, preferably two detectors (see e.g. Figs. 2A and 2B). The light sources and the detection is preferably time-controlled. The present invention is not limited to two light sources and/or two detectors. For instance, for multi-color FRET more light sources and/or more detectors may be used.

The vessel in which the biomolecule is measured can be a capillary, a microwell plate, tubing, cuvette or any other vessel.

## (A) Application of the inventive method in free solution

Preferably three fluorogenic samples are created using appropriate labelling chemistry. The samples preferably include a (first) sample that is labeled with only a donor fluorophore (preferably on the molecule of interest, or the free fluorophore may be substituted), a (second) sample that is labeled with only an acceptor fluorophore (preferably on the molecule of interest, or the free fluorophore may be substituted) and a sample that is labeled with both fluorophores.

Preferably, all three samples are preferably subjected to a "single measurement routine" at (at least one) predefined temperature, preferably in two different states (e.g. with and without ligand bound). This means preferably six samples are prepared and for each of them a single measurement is conducted. However, if a fluorophore is known to show a very low dependence of its fluorescence intensity and spectrum towards the environment, it is also conceivable to perform the single-fluorophore measurements with a fluorophore solution instead of a labeled protein. This might lead to deviations in the end result.

It is further preferred to perform a background measurement with a measurement container filled with the aqueous buffer used for the sample (ideally with and without ligand). The data is then evaluated.

## Basic description of a single measurement:

(1) The fluorogenic sample is placed in a measurement container, e.g. a glass capillary or well of a microtiter plate.
(2) The sample is illuminated as follows:

a. The sample is illuminated with excitation light of the lower (shorter) wavelength range (donor excitation). During this time the emitted light at both emission wavelength ranges is recorded (for donor and acceptor emission where these signals are termed $F_{DD}$ and $F_{DA}$, respectively).
b. The illumination is switched off. This step is preferably long enough that both detectors and the light source are properly turned off and no signal above background is recorded. This step is optional.
c. The sample is illuminated with excitation light of the higher (longer) wavelength range (acceptor excitation). During this time the emitted light at the higher (longer) emission wavelength range is recorded (acceptor emission). This signal is termed $F_{AA}$.
d. The illumination is switched off again (see (b)).
e. The cycle can be repeated several times. Note, that the order of illumination is not important and can be changed.
f. If there are several cycles, the signal of all cycles can be averaged. Measuring for several cycles can be advantageous as it allows to check if significant photobleaching occurs during the measurement time. If measurably bleaching occurs during the cycles, the information of how the balance between the different channels shifts, can be used for correction factors.

g. The duration of the illumination cycles is preferably less than 10 seconds, preferably it is in the range of 10ms to 5s, more preferably in the range of 100ms to 2s, more preferably in the range of 100ms to 1second.

## Description of the data evaluation:

(1) Preferably, the data from the illumination cycle(s) is averaged. It is further preferred to discard any data points for which the illumination and detection are not in a steady state yet.

(2) Then, every sample is preferably background-corrected by subtracting the background signal. The background signal of each channel (e.g. donor excitation-donor emission) is recorded with a filled measurement container, ideally with the same buffer that the measurement is conducted; alternatives include water. In the following, all signals have been background corrected.

(3) From the sample that contains only donor fluorescent dye, the leakage coefficient, $c_{lk}$, is calculated. This coefficient describes the fluorescent light that is emitted by the donor fluorophore, but leaks through the acceptor fluorophore filters and is thus detected as acceptor fluorescence. It is calculated by dividing the acceptor emission donor excitation channel, $F_{DA}$, by donor emission donor excitation channel, $F_{DD}$, for a donor only sample. Preferentially, this is performed with donor-only labeled biomolecule in both different binding states. But it can also be done in just one binding state or even with free dye. Still in other words, $c_{lk}$ is the ratio of the detection efficiencies of the donor fluorophore for the two different detection channels. Hence, the emission spectrum of the donor plays a role and the filter set/alignment of the setup. Still in other words, $c_{lk}$ is the ratio of the detection efficiencies of the donor fluorophore for the two different detection channels. Hence, the emission spectrum of the donor plays a role and the filter set/alignment of the setup.
Donor only sample:

$$c_{lk} = \frac{F_{DA}}{F_{DD}}$$

(4) From the sample that was labeled with only acceptor fluorescent dye, the direct excitation coefficient, $c_{dirE}$, is calculated. This coefficient describes the light emitted by the acceptor fluorophore after it was directly excited by the donor fluorophores' excitation light (without energy transfer occurring). It is calculated by dividing the acceptor emission donor excitation channel, $F_{DA}$, by the acceptor emission after acceptor excitation channel, $F_{AA}$, for an acceptor-only sample. Preferentially, this is performed with acceptor-only labeled biomolecule in both different binding states. But it can also be done in just one binding state or even with free dye. Still in other words, $c_{dirE}$ is the ratio of the excitation efficiencies of the acceptor fluorophore for the two different excitation wavelengths. Hence, the excitation spectrum of the acceptor plays a role, the filter set/alignment of the setup and the excitation intensities used.
Acceptor only sample:

$$c_{dirE} = \frac{F_{DA}}{F_{AA}}$$

(5) The sample that is labeled with both fluorophores is corrected for leakage and direct excitation. Preferentially, this correction is performed with the correction factors from the corresponding binding states.
Double labeled sample:

$$F_{DA\,corrected} = F_{DA} - c_{lk}\,F_{DD} - c_{dirE}F_{AA}$$

(6) The $\gamma$-correction factor can be calculated in at least two ways using the double labeled sample. In the first case the ratio of the differences of (a) FRET signal $F_{DA\,corrected}$, normalized by the acceptor signal of that sample and (b) donor emission after donor excitation $F_{DD}$, normalized by the acceptor signal of that sample are formed:

a.

$$\gamma = -\frac{\Delta F_{DA}}{\Delta F_{DD}}$$

with

$$\Delta F_{DA} = F_{DA}{}^{s1}/F_{AA}{}^{s1} - F_{DA}{}^{s2}/F_{AA}{}^{s2}$$

and

$$\Delta F_{DD} = F_{DD}{}^{s1}/F_{AA}{}^{s1} - F_{DD}{}^{s2}/F_{AA}{}^{s2}$$

or
with

$$\Delta F_{DA} = F_{DA}{}^{s1} - F_{DA}{}^{s2}$$

and

$$\Delta F_{DD} = F_{DD}{}^{s1} - F_{DD}{}^{s2}$$

wherein superscript "s1 and "s2" refer to the first and second condition (state 1 or condition 1; state 2 or condition 2; see formulas V-VII-2 above).

b. The difference signal is calculated between samples in different states e.g. ligand bound and no ligand bound (or two different ligands bound).

   i. To mitigate effects of different concentrations of the two samples, the data can be normalized using the acceptor emission after acceptor excitation $F_{AA}$. This is disadvantageous when the acceptor quantum yield changes significantly between the two states.
   ii. It is further preferred to use the same labeled batch for the two experiments. Note, that the data needed for calculating the $\gamma$-correction factor using two different ligand binding states does not need an additional measurement, just an additional evaluation step.

c. Alternatively, the acceptor can be partially photobleached which also leads to a difference in FRET signal and therefore to a difference in donor signal. Then state1 s1 and state2 s2 correspond to before and after photobleaching. In this case, as the same sample is used, one does not need normalization. $F_{AA}$ can be artificially set to 1 in this case to use the formulas in (a).

   i. In this case, having equal concentrations isn't a concern. However, photodamage beyond the bleaching of the red fluorophore can theoretically occur, e.g. via reactive oxygen species.
   ii. Calculating the $\gamma$-correction factor using photobleaching has the advantage that the $\gamma$-correction factor can be calculated for different biomolecule states (e.g. with and without ligand) separately. Which means, also when only one binding state can be measured.
   iii. An additional photobleaching step and an additional measurement is necessary using this type of $\gamma$-correction. However, no additional sample needs to be prepared. If the sample bleaches considerably during the aforementioned illumination cycles, this information can also be used to calculate $\gamma$.

(7) Then, the apparent FRET efficiencies of the sample with and without ligand (or with two different ligands) can be calculated from the double-labeled sample by using the corrected $F_{DA}$ signal and the gamma correction factor:
Double labeled sample:

$$E_{app} = \frac{F_{DA\ corrected}}{\gamma F_{DD} + F_{DA\ corrected}} = E_{real}\frac{c_{DA}}{c_{DA} + c_D}$$

$c_{DA}$ and $c_D$ are the concentrations of the double-labeled biomolecule and the only donor labeled biomolecule, respectively.

(8) From there, the ratio of real FRET efficiencies is calculated by using:

$$\frac{E_{app1}}{E_{app2}} = \frac{E_{real1}}{E_{real2}}$$

With $E_{app}$ being the apparent FRET efficiency as calculated above and $E_{real}$ is the real efficiency of the energy transfer, a physical property of the labeled molecule in question.

(9) The distance of the inter-fluorophore distance r1 can be calculated using the Förster radius $R_0$ and one known distance $r_2$:

$$E_{real} = \frac{R_0^6}{r^6 + R_0^6}$$

$$\frac{E_{app2}}{E_{app1}} = \frac{r_1^6 + R_0^6}{r_2^6 + R_0^6}$$

$$r_1 = \sqrt[6]{\frac{E_{app2}}{E_{app1}}\left(r_2^6 + R_0^6\right) - R_0^6}$$

This calculation can be performed where the Förster radius $R_0$ does not change with ligand binding. This is the case if the refractive index of the medium, the emission spectrum of the donor, the excitation spectrum of the acceptor, the fluorophore orientation and the donor quantum yield do not change upon ligand binding. Out of these, the quantum yield of the donor and the fluorophore orientation are the most likely to change.

**(B) Application of the inventive methods in which the labelled biomolecule is at the outer surface of a cell**

The method of the present invention can also be used in case a labelled biomolecule is at the outer surface of a cell, e.g., on the outer surface of eukariotic, prokaryotic, archea, bacteria, human cells or yeast cells. The method steps are similar to the above discussed case were proteins are in free solution.

**Brief description of a measurement sequence:**

Three fluorogenic samples are created using appropriate labelling chemistry, biochemistry or molecular biology method (e.g. plasmid to express a protein with GFP). The samples preferably include a sample that is labeled/expressed with only a donor fluorophore/fusion protein (preferably on the molecule of interest, or the free fluorophore may be substituted), a sample that is labeled/expressed with only an acceptor fluorophore/fusion protein (preferably on the molecule of interest, or the free fluorophore may be substituted) and a sample that is labeled/expressed with both fluorophores/fusion proteins.

Preferably, all three samples are subjected to the "single measurement routine" at (at least one) predefined temperature, preferably in two different states (e.g. with and without ligand bound). This means preferably six samples are prepared and for each of them a single measurement is conducted. Again, if a fluorophore is known to show a very low dependence of its fluorescence intensity and spectrum towards the environment, it is conceivable to perform the single-fluorophore measurements with a fluorophore solution instead of a labeled protein. This might lead to deviations in the end result. Only four or five measurements are performed in this case.

Additionally, a background measurement is preferably performed with a measurement container filled with the aqueous buffer used for the sample (ideally with and without ligand). The data is then evaluated.

**Basic description of a single measurement:**

(1) The fluorogenic sample is placed in the measurement container, e.g. a glass capillary or well of a microtiter plate.

(2) The sample is illuminated as follows:

a. The sample is illuminated with excitation light of the lower wavelength range (donor excitation). During this time the emitted light at both emission wavelength ranges is recorded (for donor and acceptor emission where these signals are termed $F_{DD}$ and $F_{DA}$, respectively).
b. The illumination is switched off. This step is preferably long enough that both detectors and the light source are properly turned off and no signal above background is recorded. This step is optional.
c. The sample is illuminated with excitation light of the higher wavelength range (acceptor excitation). During this time the emitted light at the higher emission wavelength range is recorded (acceptor emission). This signal is termed $F_{AA}$.
d. The illumination is switched off again (see (b)).
e. The cycle can be repeated several times. Note, that the order of illumination is not important and can be switched.
f. If there are several cycles, the signal of all cycles can be averaged. Measuring for several cycles can be advantageous as it allows to check if significant photobleaching occurs during the measurement time. If measurably bleaching occurs during the cycles, the information of how the balance between the different channels shifts, can be used for correction factors.
g. the duration of the illumination cycles is preferably less than 10 seconds, preferably it is in the range of 10ms to 5s, more preferably in the range of 100ms to 2s, more prefarbly in the range of 100ms to 1second.

The data evaluation is preferraly similar or even identical to the above discussed case were the proteins are provided in free solution.

Examples

Methods and materials are described herein for use in the present disclosure; other, suitable methods and materials known in the art can also be used. The materials, methods, and examples are illustrative only and not intended to be limiting.

I. Background

The experiments described in the following were exemplarily performed using a maltose-binding protein and a substrate-binding-domain protein. The maltose-binding protein (MalE or MBP) from *Escherichia coli* and the substrate-binding-domain protein (SBD) from *Lactococcus lactis* are proteins that are responsible for nutrient uptake. These proteins do not have any endogenous cysteine. However, by targeted exchange of an endogenous amino acid positioned at a surface of the protein with a cysteine, the respective protein can specifically be fluorophore-labeled via said cysteine(s) using, e.g., maleimide chemistry.

MalE is a periplasmic transporter protein involved in the sugar supply of the organism. It binds maltose and its derivatives, subsequently transferring it to a membrane transporter, which transfers the sugar into the cytoplasm of the cell. Herein, two mutants of MalE were used, i.e. MalE47 with a A141C-T36C mutation in the protein backbone, and MalE50 with a S353C-L29C mutation close to a binding pocket. In case of MalE47, the distance between the two mutations A141C and T36C increased from 3.6 nm in the open conformation (apo conformation, i.e. without sugar bound as ligand) to 4.2 nm in the closed conformation (holo conformation, i.e. with sugar bound as ligand; **Figure 3 A).** In case of MalE50, the distance between the two mutations S353C and L29C decreased from 6.1 nm in the open conformation to 5.2 nm in the closed conformation **(Figure 3 B).**

SBD is involved in the transport of glutamine and asparagine into the cell. SBDs are anchored to ABC importers in the cell membrane. The SBD used herein consisted of two fully functional SBD subunits (SBD1 and SBD2) fused together. SBD1 can bind glutamine and asparagine, whereas SBD2 binds glutamine only. More specifically, SBD2-1 was used, a T369C-S451 C mutant of SBD2 fused to a SBD1 subunit, wherein the mutations T369C and S451C were located close to a binding pocket. As in case of MalE50, the distance between the two mutations decreased from 4.9 nm in the open conformation to 4 nm in the closed conformation (**Figure 3 C**).

II. Materials

In the following, non-limiting examples of materials are briefly listed which were used in certain examples for conducting the method of the present invention.

1. Chemicals

| Name (Catalog No.) | Supplier |
|---|---|
| Bovine Serum Albumin Fraction V, fatty acid free (A8806-5G) | Sigma Aldrich |
| GE Ni-Sepharose 6 Fast Flow (17-5318) | GE Healthcare |
| Glycerin, ROTIPURAN, min. 99,5 % (3783.2) | Carl Roth |
| Imidazol, min. 99 % (3899.2) | Carl Roth |
| Kaliumchlorid, min. 99,5 % (6781.3) | Carl Roth |
| **L-GLUTAMIN 200 MM SOLUTION (G7513-100ML)** | Sigma Aldrich |
| Maltose monohydrate (1059100500) | Sigma Aldrich |
| **PHOSPHATE BUFFERED SALINE, 7.4, KALIUMFREI, TABLETTEN (1113.1)** | Carl Roth |
| HCL (9787.1) | Carl Roth |
| TRIS-HCl (9090.2) | Carl Roth |
| Pluronic®F-127 (9003-11-6) | Sigma Aldrich |

2. Consumables

| | Name (Catalog No.) | Supplier |
|---|---|---|
| **Fluorophores** | Alexa Fluor™546 C5 Maleimide (A10258) | Thermo Fischer Scientific |
| | Alexa Fluor™647 C2 Maleimide (A20347) | Thermo Fischer Scientific |
| **Sample holder** | Monolith NT.115 Premium Capillaries (MO-K025) | Nanotemper technologies |

| | High Precision Microscope Cover Glasses (0107242) | Marienfeld |
|---|---|---|
| **Reaction Tubes** | MProtein LoBind Tubes (0030108094) | Eppendorf |
| **Column** | Poly-Prep Chromatography column | Bio-Rad |

*3. Buffers*

| Name | Composition |
|---|---|
| PL1 | 50 mM Tris-HCl pH 7.4, 50 mM KCl |
| PL2 | 50 mM Tris-HCl pH 7.4, 50 mM KCl, 50% Glycerol |
| PL3 | 50 mM Tris-HCl pH 7.4, 50 mM KCl, 500mM Imidazol, 5% Glycerol |
| Imaging Buffer | 50 mM Tris-HCl pH 7.4, 50 mM KCl, 0.1% Pluronic |
| Holo | 50 mM Tris-HCl pH 7.4, 50 mM KCl, 0.1% Pluronic, 2 mM Maltose |
| Apo | 50 mM Tris-HCl pH 7.4, 50 mM KCl, 0.1% Pluronic |

III. Methods

*1. Molecules and labelling*

His-tagged MalE and SBD proteins were labelled with Alexa 546 (donor fluorophore; D) and Alexa 647 (acceptor fluorophore; A) dye, respectively, using maleimide chemistry. Briefly, about 100 ng protein, 49 $\mu$l PL1 buffer and 1$\mu$l DTT (1M) were incubated on ice for 30-60 minutes for reducing cysteines. A Bio-Rad Poly-Prep Chromatography column was washed two times with milliQ water, followed by the placement of 160 $\mu$l Ni Sepharose 6 Fast Flow resin on the column's membrane. The ethanol contained in the resin was washed away with two column volumes (CV) milliQ water. The resin was subsequently equilibrated with two CV PL1 buffer. Approximately 1 ml buffer remained in the column before proteins were added to the column and fixed by the resin, followed by a washing step for removing remaining DTT with two CV of PL1 buffer. Approximately 0.5 ml buffer was left in the column. For dye preparation, 25 nmol Alexa 647 and 80 nmol Alexa 546 were diluted in 5 $\mu$l DMSO for A- and D-labelling, and for A-only and D-only labelling 25 nmol of the respective dye was diluted in 5 $\mu$l DMSO. The respective dye mixture was added to the proteins in the column and mixed by pipetting gently up and down. The column was sealed with parafilm and incubated over night at 4 °C. Remaining dyes were removed by a washing step using one CV PL1 followed by two CV PL2 buffer. Labelled proteins were eluted with 500 $\mu$l PL3 buffer, collected in a low binding test tube, and purified by size-exclusion chromatography to remove remaining imidazole, glycerol and fluorophores.

Thus, for a respective protein A-only labelled proteins, D-only labelled proteins, and A- and D-labelled proteins were obtained; also referred to in the following as respective A-labelled proteins, D-labelled proteins, and AD-labelled proteins.

*2. smFRET*

*2.1 Sample handling*

Labeled proteins were diluted in imaging buffer to a final concentration of 200 pM (stock solution). The object slide was passivated for 2 minutes with 100 $\mu$l 1 mg/ml BSA in PBS buffer and the remains discarded afterwards with a pipette. The protein stock solution was mixed 1:1 with either the Holo or Apo buffer followed by the placement of a 100 μl droplet on the passivated object slide.

*2.2 Data acquisition*

The subsamples were measured in alternating laser excitation mode (ALEX) using a confocal microscope with a duration of 50 μs per light source. The light sources were 532 and 640 nm cw-lasers with a laser power set at 60 mW and 25 mW, respectively, to ensure a stoichiometry S of approximately 0.5. Three intensity time traces were recorded $(F_{DA}, F_{DD}, \text{and } F_{AA})$.

*2.3 Burst search*

The software ALEX-suite comprises two burst search algorithms for FRET analyses. The All Photon Burst Search (APBS) algorithm was used to calculate FRET efficiencies and S of D-only and A-only labelled proteins (Eggeling et al., 1998, PNAS, 95(4):1556-1561). This enabled the determination of leakage and direct excitation coefficients. Furthermore, the Dual Channel Burst Search (DCBS) algorithm was used to calculate FRET efficiencies for AD-labelled proteins by applying the respective determined correction factors (Nir et al., 2006, J PHYS CHEM B, 110(44):22103-22124). The DCBS algorithm was also used for the estimation of γ-correction factors.

*2.4 Background correction*

Background correction was done according to Hohlbein et al. (2014; Chemical Society Reviews, 43(4):1156-1171). Briefly, background intensities were estimated by calculating the mean count rate for $F_{DA}$, $F_{DD}$, and $F_{AA}$, respectively, if the total photon count was less than 10 counts per ms. This mean count rate was multiplied by the length of each burst and the result subtracted from the signal.

*2.5 Leakage and direct excitation coefficients*

Leakage and direct excitation coefficients were determined according to Hohlbein et al. (2014; CHEM SOC REV, 43(4):1156-1171) by applying a threshold for D-only and A-only labelled proteins. For D-only labelled proteins the threshold was set to S > 0.95 and for A-only E > 0.85 and S < 0.2. A one-dimensional Gaussian curve was fitted over the D-only and A-only labelled proteins. By estimating the peak of each Gaussian curve ($\mu$), leakage and direct excitation coefficients were estimated as follows: correction factor = $\mu / (1 - \mu)$. $F_{DA}$ was corrected as described above to obtain $F_{DAcor}$.

*2.6 γ-correction factor*

S and the E peaks (from apo and holo measurements) were used to determine respective γ-correction factors. Therefore, apo and holo measurements were corrected for background and crosstalk effects. Then, by plotting $1/S$ against $E$ from both measurements and fitting a linear function between the measurements, the slope ($\Sigma$) and the intercept ($\Omega$) were obtained. The γ-correction factor was subsequently calculated as $\gamma = (\Omega - 1)/(\Omega + \Sigma - 1)$, and then $E_{app}$ was calculated as described above.

*3. eFRET*

*3.1 Sample handling*

Labeled proteins were diluted in imaging buffer to a final concentration of 100 nM (stock solution). The protein stock solution and the Apo buffer were mixed in a 1:1 ratio and mixed by pipetting gently up and down. Monolith Premium Capillaries were filled with the mix and put into the eFRET-device's tray occupying the positions 1 to 8. As regards the Holo buffer, the same applied, except that positions 9 to 16 were occupied.

*3.2 Data acquisition*

The samples were measured in an alternating excitation mode on an eFRET device. The light sources were provided by LEDs set to 15 mA for the green LED and 3.5 mA for the red LED. The capillary scan was conducted using $F_{DD}$ by scanning each capillary for their x and z position. The respective position with the highest fluorescence in both axes was saved for the measurement. The measuring time for each capillary was set to last for 2 seconds with a sampling rate of 4,000 data points per second. The alternating excitation cycle was set to 25 ms for each light source with a resting period in-between for 1 ms. The three fluorescence intensities were recorded: $F_{DA}$, $F_{DD}$, and $F_{AA}$. Here, the first 10 ms and the last 5 ms of the respective recorded signals were excluded to ensure a stable signal. The 400 data points coming from the respective remaining 10 ms signal were averaged and stored for $F_{DA}$, $F_{DD}$, and $F_{AA}$, respectively.

### 3.3 Background correction

The background signal intensity was estimated by measuring imaging buffer only. No capillary scan was performed since this might have affected the estimation of the capillary coordinates. Therefore, the positions from previous eFRET-measurements were used. Measured background signals obtained for $F_{DA}$, $F_{DD}$, and $F_{AA}$ were stored for each capillary and subsequently subtracted from respective FRET-measurements.

### 3.4 Leakage and direct excitation coefficients

The leakage coefficient $c_{lk}$ was estimated by measuring respective D-labeled proteins. After a capillary scan, signals obtained for $F_{DA}$, $F_{DD}$, and $F_{AA}$, respectively, were stored for each capillary. After background correction, $c_{lk}$ was calculated for each capillary as described above.
The direct excitation coefficient $c_{dirE}$ was estimated by measuring respective A-labeled proteins. No capillary scan was conducted since this was automatically done using $F_{DD}$. Saved positions of previous measurements were used (i.e. the respective measurement used for estimation of $c_{lk}$). After background and leakage correction, $c_{dirE}$ was calculated for each capillary as described above.

### 3.5 $\gamma$-correction factor

The $\gamma$-correction factor was determined as described above using signals corrected for resepctive background and cross-talk effects. A normalization step was applied, wherein $F_{DA}$ and $F_{DDcor}$ *were* divided by $F_{AAcor}$ capillary wise to obtain *norm*$F_{DA}$ and *norm*$F_{DDcor}$ signals for apo and holo (capillaries 1-8 and 9-16, respectively), which were then averaged, followed by the calculation of the deltas.

### 3.6 $E_{app}$ ratio

$E_{app}$ and $E_{app}$ ratios were determined as described above. $E_{app}$ was either calculated using the non-normalized $\gamma$-correction factor or the normalized $\gamma$-correction factor, wherein the latter resulted in $_{norm}E_{app}$ and a respective $_{norm}E_{app}$ ratio.

### 3.7 D-only titration

A two-step titration was done using D-labelled proteins. Three vessels were used, of which the first one contained D-only labelled protein diluted in imaging buffer at a concentration of 100 nM. From there a sample was taken and mixed into the second vessel with imaging buffer at a 1:1 ratio resulting in a total concentration of 50 nM. This step was repeated for the third vessel resulting in a final concentration of 25 nM. The respective content of the three vessels was then mixed at a 1:1 ratio with 100 nM DA-labelled proteins, resulting in a final protein concentration of 200 nM for the first vessel; 150 nM for the second vessel and 125 for the third vessel. Then, all obtained protein concentrations were equalized via dilution to a final protein concentration of 100 nM. For comparison, a fourth vessel containing 100 nM of DA-labelled protein was used. The respective content of these four vessels is referred to in the following also as respective protein stock solution (cf. above).

### 4. smFRET data conversion into eFRET data

The output of smFRET measurements consisted of an enumeration of recorded bursts with their respective meta-information, such as E and S values, burst length and photon counts detected for $F_{DA}$, $F_{DD}$, and $F_{AA}$, respectively. The conversion of smFRET data into eFRET data was done by removing information not present in eFRET measurements, such as S values, burst lengths, etc .... Thus, information remaining in eFRET data was information from $F_{DA}$, $F_{DD}$, and $F_{AA}$. As eFRET measurements cannot distinguish single burst events, all photon counts of each burst detected for $F_{DA}$, $F_{DD}$, and $F_{AA}$, respectively, were added. Hence, E and S values of detected bursts were removed and the respective fluorescence signal partitioned into $F_{DA}$, $F_{DD}$, and $F_{AA}$, respectively.

IV. Results and discussion

A plurality of experiments showed that the corrections of the present invention work and provide reliable results. In particular, in certain examples (1) a double-labeled sample, (2) a donor only labeled sample and (3) an acceptor only labeled sample were measured in a state-of-the-art single molecule setup (smFRET). By summing up the intensities measured in the single molecule setup, ensemble data could be simulated. With that data, the specific correction factors for this setup (excitation power, filter sets, optical alignment) could be calculated, while at the same time the exact same data set to analyze the data in the state-of-the-art single molecule fashion was used.

For instance, the energy transfer efficiency ratio of substrate binding domain 2-1 from Lactococcus lactis (SBD2-1) with and without ligand was measured to be 1.445 with accurate smFRET and 1.438 using the data pipeline of the present invention with the exact same data summed up (see e.g. Fig. 4). The difference is less than 0.5% and can be attributed to noise and effects of bleaching, which is more common in a setup with a high laser power such as a smFRET setup, than a setup constructed to measure ensemble fluorescence. The occurrence of bleaching during the diffusion time of single molecules through the confocal laser spot shows as a straight line between the FRET events (Fig. 4A, two rightmost E-S histograms, middle) and donor only events (same panels, upper left corner). In particular, Fig. 4 shows the following:

(A): Single molecule data in the very common S-E histogram view. In this view the energy transfer efficiency is on the x-axis and the stochiometry is plotted on the y-axis.

In (B) and (C), the data was added up to yield intensity bins of different filters, which can then be put through the same data pipeline as ensemble data.

(D) shows the FRET efficiency histograms of the data shown in (A), with the dotted red lines representing the mean of the distribution.

Moreover, three different proteins (SBD2-1, MalE-50 and MalE47) were measured in a functional demonstrator eFRET setup which collects whole datasets of many samples within seconds to minutes. The solutions with and without ligand were placed in glass capillaries which were illuminated with LEDs of green and red light. The emission light was divided by a dichromatic mirror and recorded using two detectors (see e.g. 2B). The energy transfer ratio was quantified by using the full set of corrections discussed earlier and compared with the result from smFRET. The data deviated by 4% to 6% compared to smFRET. This is in the range that state-of-the-art smFRET setups differ (with higher energy transfer efficiency correlating with lower standard deviations; see e.g. Doi: 10.1038/s41592-018-0085-0).

|  | Single molecule | | | Ensemble measurement | | |
|---|---|---|---|---|---|---|
|  | No ligand | With ligand | Ratio | No ligand | With ligand | Ratio |
| SBD2-1 (replicate) | 0.526 | 0.752 | 1.43 | 0.219 | 0.293 | 1.33 |

| MalE47 (replicate) | 0.856 | 0.747 | 0.87 | 0.188 | 0.166 | 0.88 |
| MalE50 (replicate) | 0.444 | 0.605 | 1.36 | 0.107 | 0.139 | 1.30 |

Table 1

Table 1 shows the comparison of single molecule FRET (smFRET) efficiencies with ensemble measurements (eFRET or quantumFRET) using the corrections that are part of the present invention. While the apparent energy transfer efficiencies differ quite significantly due to the presence of donor-only labeled proteins, the ratio of efficiencies is very well within the accuracy of smFRET measurements, while taking only seconds compared to 1-2h for the single molecule data.

Further experiments demonstrate that the method of the present invention leads to robust energy transfer efficiency ratios, independent of labeling efficiency and potential contributions of donor-only labeled biomolecule, something that other ensemble methods do not address. In particular, two previously characterized protein constructs MalE47 and MalE50 were used and added varying amounts of donor-only labeled proteins to an (already incompletely) double-labeled sample. While the corrected energy efficiencies changed dramatically with a decreasing ratio $c_{DA}/(c_{DA} + c_D)$ down to half its original value (corrected for background, crosstalk (leakage and direct excitation) and brightness (y)) (Fig.11), it was shown that the energy transfer efficiency *ratio* remains constant and is thus a quantitative measure that does not depend on labeling efficiency (Fig.12). The variation between measurements was minimal and potentially lower than smFRET measurements.

In particular, Fig. 11 illustrates the corrected energy transfer efficiencies which are influenced by the amount of donor-only labeled protein for MalE47 (upper panel) and MalE50 (lower panel). Both proteins are maltose binding proteins with specific mutations. Apo stands for the proteins without maltose, so in an empty conformation, while holo stands for the proteins with maltose bound. As expected, the apparent energy transfer efficiencies decrease to roughly half as the ratio of correctly labeled protein is decreased to half (compare pure sample with 1:1 dilution).

Fig. 12 shows the energy efficiency ratios, which are independent of the amount of donor-only labeled molecules present for MalE47 (left panel) and MalE50 (right panel). These ratios are the same data that is shown in Fig. 10

### 1. Experimental validation of eFRET-correction-factors using smFRET

#### 1. 1 Leakage coefficient $c_{lk}$

Data obtained from smFRET measurements were converted into eFRET data. smFRET and eFRET data were background-corrected by subtracting respective background signals. Obtained $c_{lk}$ had a mean value of 0.067 for eFRET **(new Figure 4-2 A**, left panel), and a mean value of 0.059 for smFRET **(Figure 4-2 A,** mid-left panel).

For smFRET, $c_{lk}$ values were comparable with 0.0651 for DA-labelled proteins, 0.0618 for A-labelled proteins, 0.0601 for D-labelled proteins, and 0.0619 for background. Thus, $c_{lk}$ obtained from the D-labelled proteins was chosen as the leakage coefficient to be used in the following (i.e. also for A-labelled proteins) due to its high signal density compared to the background, its accuracy compared to smFRET data and the comparable results obtained for the different $c_{lk}$ values.

#### 1.2 Direct excitation coefficient $c_{dirE}$

$c_{dirE}$ was obtained using a Channel-S-plot (ChS). For A-only labelled and background measurements, a signal was detected for $F_{DD}$ that contributed to leakage observed for $F_{DA}$. Consequently, the signal source in $F_{DA}$ appears to be a mixture of direct excitation and leakage. In comparison, the ChS plot for D-only labelled proteins did not show any signal mixture. Consequently, $c_{dirE}$ determination should comprise a correction for leakage. Thus, the corrected calculation of $c_{dirE}$ was used as described above. This resulted in a deviation of $c_{dirE}$ of 3% compared to the real $c_{dirE}$ (*real_dir*; **new Figure 4 A,** mid-left and left panel) and thus, a difference between the two estimated coefficients as in case of the $c_{lk}$ values described in the section above.

#### 1.3 y-correction factor

The *γ-correction* factor was obtained based on the measurements of the DA-labelled proteins in their respective open and closed conformation corrected for leakage and direct excitation. In general, obtaining the *γ-correction* factor based on differences between $F_{DAcor}$ and $F_{DDcor}$ can be highly affected by differences in the exact protein concentration between apo and holo measurements. Hence, for diminishing potential effects of concentration differences, a normalization step was performed. Therefore, $F_{DDcor}$ and $F_{DAcor}$ were divided by $F_{AA}$ as $F_{AA}$ did not record any FRET event and thus, was apparently a good indicator for such concentration differences.

*γ-correction* factors obtained for smFRET and eFRET exhibited a comparable difference of approximately 27%. No significant improvement in *γ-correction* factors (gamma*; improvement of 0.3%; **new Figure 4 B)** was observed upon applying correction factors obtained from smFRET to eFRET data. Thus, the observed variance was not significantly affected by differences in cross-talk correction factors used in smFRET and eFRET approaches.

#### 1.4 $E_{app}$ ratio

Both *γ-correction* factors were used to determine $E_{app}$ of open and closed conformation, respectively. The resulting values were about one fifth of the respective smFRET $E_{app}$ values. Obtained $E_{app}$ ratios revealed an approximately 3% difference between smFRET and eFRET approaches **(new Figure 4 C).**

Furthermore, the influence of the different *γ-correction* factors was tested. Thus, the *γ-correction* factors obtained from smFRET were used for determining eFRET $E_{app}$ ratios (E-ratio*; **new Figure 4 C).** However, E-ratio* did not show a significant improvement compared to the $E_{app}$ ratio from eFRET, supporting the robustness of the eFRET approach disclosed herein.

*1.5 Discussion*

The results obtained using the eFRET approach showed a comparable variation as in case of smFRET. However, differences in the $\gamma$-correction factor between replicates in smFRET were considerable. This might have resulted, e.g., from the following two reasons. First, smFRET uses an approach based on *E* and *S* peaks obtained from apo and holo measurements, which is sensitive even to minimal signal changes. Furthermore, the measured FRET peaks were not Gaussian shaped, which might have further affected the determination of the $\gamma$-correction factor. Second, smFRET requires a pico molar solution of a given protein. Thus, smFRET can be easily affected by background noise. In contrast, eFRET measurements require a higher, e.g. nano molar, protein concentration and thus, any background contribution is less predominant and comparatively easy to estimate using, e.g., a water sample - an approach that cannot be used in case of smFRET.

*2. eFRET data acquisition and processing*

*2.1 Raw data*

The raw data supported the expected behavior of the proteins: SBD2-1 and MalE50 were designed to show an increase in their respective $E_{app}$ from the open to the closed conformation **(Figure 3)**. This was reflected by a loss of $F_{DD}$, a gain of $F_{DA}$, and thus, a raise in $E_{app}$. In contrast, MalE47 was designed to exhibit a decrease in $E_{app}$ from the open to the closed conformation. Correspondingly, a gain of $F_{DD}$, a loss of $F_{DA}$, and thus, a loss in $E_{app}$ was observed. Again, $F_{AA}$ could be used as a quality control as the recorded signals did not show a significant difference between the apo and holo measurements. Of note, SBD2-1 was the only exception showing a significant difference in $F_{AA}$ between the apo and holo measurements. However, said signal difference diminished once $E_{app}$ was calculated. This observation was supported by high Z-factors, which were 0.97 for SBD2-1, 0.925 for MalE50 and 0.89 for MalE47.

*2.2 Background and cross-talk correction factors*

Cross-talk and background correction steps were conducted capillary wise to take capillary position specific signal patterns and/or concentration differences into account.
MalE50 and SBD2-1 revealed a slightly different $c_{lk}$ between the two conformations in contrast to MalE47. Thus, a conformation dependent correction factor appeared to be advantageous, i.e. apo being corrected with the respective apo correction factor and holo being corrected with the respective holo correction factor.
MalE47 showed a significant difference of $c_{dirE}$ between the two conformations, whereas results obtained for SBD2-1 were ambiguous. However, MalE50 did not show a significant difference of $c_{dirE}$ between apo and holo.
Overall, differences in leakage and direct excitation coefficients were less than 0.5% and should not significantly affect the correction of $F_{DA}$.

*2.3 Correction & normalization of $F_{DD}$ and $F_{DA}$*

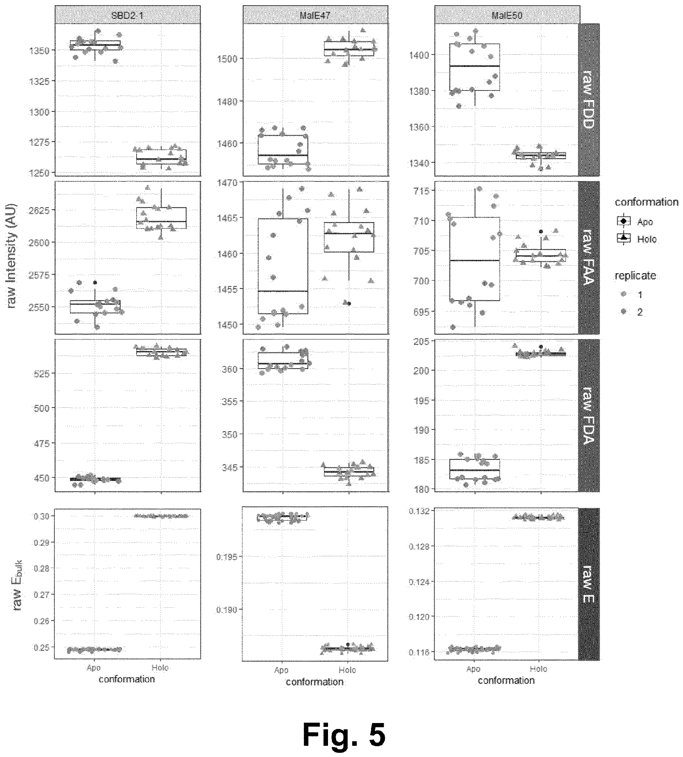

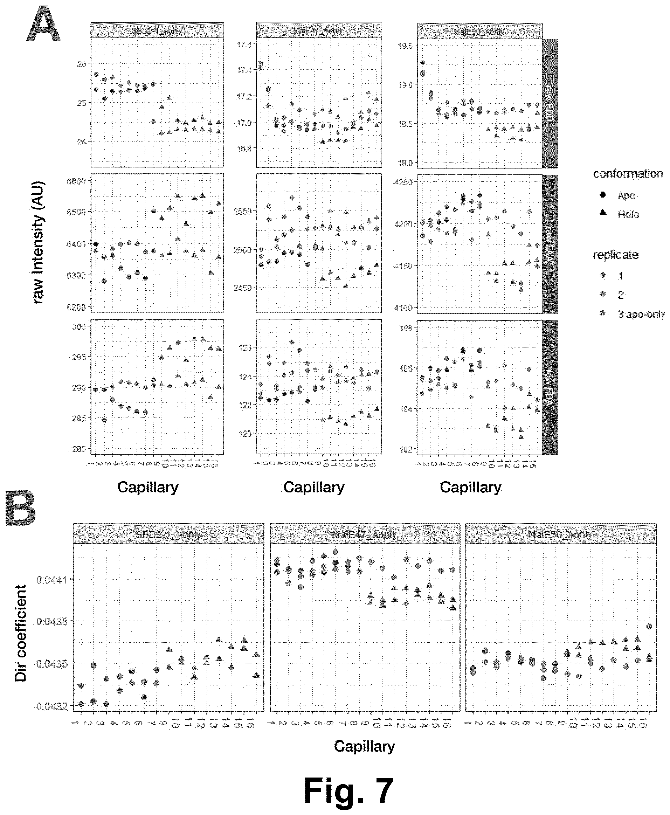

The determination of the $\gamma$-correction factor requires a background corrected $F_{DD}$, as well as an $F_{DA}$ corrected for cross-talk and background effects. In **Figure 7,** raw values detected for $F_{DA}$, $F_{DD}$, and $F_{AA}$ are shown.
Non-normalized $F_{DA}$ had a reduced variance compared to $F_{DA}$ obtained upon smFRET data conversion **(Figure 5)** due to the applied cross-talk and background corrections **(Figure 8)**. On the other hand, $F_{DDcor}$ still showed a notable variance among the replicates after background-correction (cf. e.g. MalE50 measurement, wherein the variance for apo is much larger than for holo). It could be expected that the resulting $\gamma$-correction factors would be significantly different among the replicates due to $F_{DDcor}$ required for their determination. However, though said signal difference was partially compensated after the $F_{AAcor}$ normalization step, it still showed a considerable variance compared to the normalized $F_{DA}$ as only a background-correction step was applied to *FDDcor*.
Thus, a multiple-step correction applied to $F_{DA}$ is highly advantageous compared to a single-step correction for background-correction only.

*2.4 accurate FRET-efficiency-ratios*

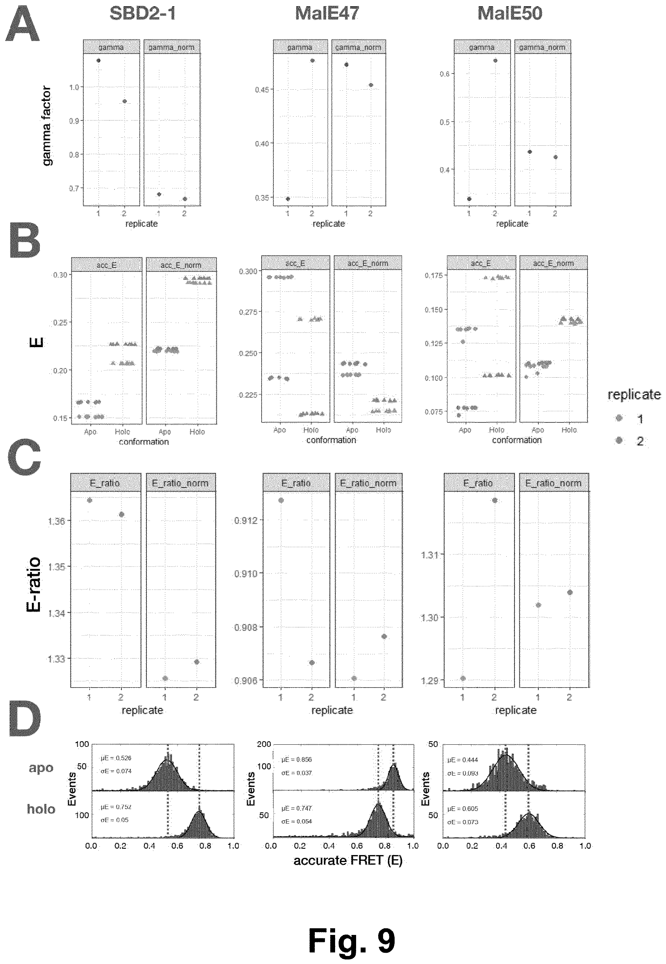

$\gamma$-correction factors were calculated using non-normalized and normalized $F_{DDcor}$ and $F_{DA}$ and their contribution to $E_{app}$ and $E_{app}$ ratios compared.
$\gamma$-correction factors from non-normalized $F_{DA}$ and $F_{DD}$ showed a higher difference among replicates compared

to $\gamma$-*correction* factors from normalized $F_{DA}$ and $F_{DD}$ **(Figure 9)**. Consequently, also non-normalized $E_{app}$ showed a greater difference between replicates, whereas $E_{app}$ obtained upon smFRET data conversion did not show a significant difference among replicates **(Figure 5)**.

Normalized $F_{DA}$ and $F_{DD}$ did not show a significant variance among replicates, resulting in comparable $\gamma$-*correction* factors **(Figure 8),** though affecting $E_{app}$. However, these differences apparently had only a minor impact on the determination of the $E_{app}$ ratios. For example, MalE47 having the biggest variance among its normalized $E_{app}$ values exhibited $E_{app}$ ratios with only about 0.2% difference between replicates. Also normalized $E_{app}$ ratios of SBD2-1 and MalE47 had only about 0.2% and 0.1% difference between replicates, respectively.

Importantly, the normalized $E_{app}$ ratios were comparable to the $E_{app}$ ratios obtained with smFRET with the differences between being about 6%, 4% and 4% for SBD2-1, MalE47 and MalE50, respectively, which is in the same range as results from different smFRET labs differ when measuring the exact same sample.

*3 Validation of eFRET-measurements robustness*

As the determination for FRET-efficiencies depends on the D-only labelled molecules of the FRET sample, a D-only titration was used to determine impact on the $\gamma$-*correction* factors and the resulting $E_{app}$ and $E_{app}$ ratios.

The obtained $E_{app}$ values increased as the concentration of the D-only labelled protein decreased **(Figure 10)**. This was not observed in case of the non-normalized $E_{app}$ as the determination of the $\gamma$-*correction* factor can be sensitive to differences in concentrations. However, normalized $E_{app}$ increased as the concentration of the D-only labelled protein decreased due to comparable normalized $\gamma$-*correction* factors (e.g. error of 16% for MalE47 and 18% for MalE50). In comparison, the non-normalized $\gamma$-correction factors had an error up to 80%.

Importantly, obtained normalized $E_{app}$ ratios were comparable with the values having an error of less than 1 % for MalE50 and 0.8% for MalE47 for example. Hence, the normalized $E_{app}$ ratios were consistent and reproducible. However, the eFRET $E_{app}$ ratios had a consistent error compared to the smFRET $E_{app}$ ratios of approximately 5%. This error was not due to the D-only labelled proteins as the D-only titration experiment revealed similar $E_{app}$ ratios, though still with a comparable 5% error. Instead, said error might originate from hardware components of the device, and/or biophysical effects that were not considered such as fluorophore bleaching.

Two use-cases can be distinguished for protein-protein interactions: (1) naturally occurring protein-protein interactions for which an inhibitor is wanted, (2) naturally not occurring or only weakly occurring interactions that should be strengthened or created de-novo, e.g. through PROTACs. In both cases eFRET and $E_{real}$ can be used for characterization. With the ability to mitigate the effects of background, leakage and direct excitation, as well as unequal fluorophore brightness (y), $E_{real}$ is subject to significantly less noise that other ensemble FRET methods such as alphaScreen. It has also been shown, that different PROTACs link proteins more loosely (the proteins are not in close contact) or more tightly (the PROTAC strengthens bonds on the protein surface). Such differences are expected to show in different $E_{real}$ values.

V. Conclusion

Accurate eFRET, as demonstrated herein, represents a robust approach for measuring inter- and intra-protein conformation and can be used for high-throughput screening to identify new drug candidates and/or to characterize conformational changes of a protein under different conditions such as in the presence of a ligand.

**Claims**

1. A method for obtaining quantitative information on average donor-acceptor distance changes within a molecule using ensemble Förster resonance energy transfer (eFRET), the method comprising the steps of:

   - obtaining a donor sample, wherein said donor sample comprising at least a donor and/or donor-labelled copies of said molecule;
   - obtaining an acceptor sample comprising at least an acceptor and/or acceptor-labelled copies of said molecule; and
   - obtaining at least a donor-acceptor-labelled sample comprising donor-acceptor-labelled copies of said molecule;
   - performing an eFRET measurement for the donor-acceptor-labelled sample under a first condition and a second condition to obtain for this samples a first eFRET result under the first condition and a second eFRET result under the second condition, wherein the eFRET measurements of the donor-acceptor-labelled sample are performed using multiple respective molecule copies;

- performing at least an eFRET measurement for the donor sample under at least one condition to obtain for this sample a first eFRET result and calculating a leakage coefficient ($c_{lk}$) from said donor sample;
- performing at least an eFRET measurement for the acceptor sample under at least one condition to obtain for this sample a first eFRET result and calculating a direct excitation coefficient ($c_{dirE}$);
- correcting the donor-acceptor-labelled eFRET results based on the leakage coefficient ($c_{lk}$) and the direct excitation coefficient ($c_{dirE}$);

wherein the first and/or the second eFRET results comprise respective measurement values of the

- emission of the acceptor after donor excitation ($F_{DA}^{(A)}$),
- emission of the acceptor after acceptor excitation ($F_{AA}^{(A)}$), and/or
- emission of the donor after donor excitation ($F_{DD}^{(D)}$),
- emission of the donor after acceptor excitation ($F_{DA}^{(D)}$)

wherein said leackage coefficient ($c_{lk}$) is determined based on the ratio of $F_{DA}^{(D)}$ and $F_{DD}^{(D)}$; and said direct excitation coefficient ($c_{dirE}$) is determined based on the ratio of $F_{DA}^{(A)}$ and $F_{AA}^{(A)}$;

- calculating a $\gamma$-correction factor based on the donor-acceptor-labelled sample(s), wherein the $\gamma$-correction factor is based on the negative ratio of i) the difference of $F_{DA}$ between the first and the second condition and ii) the difference of $F_{DD}$ of the donor-acceptor-labelled sample between the first and the second condition; or calculating the $\gamma$-correction factor using a photobleaching method in which the acceptor is partially photobleached which leads to a difference in FRET signal and therefore to a difference in donor signal;
- determining condition-specific eFRET efficiencies based on the corrected donor-acceptor-labelled eFRET results and by using the $\gamma$-correction factor; and
- determining quantitative information on average donor-acceptor distance changes within the molecule under the first and the second condition using the condition-specific eFRET efficiencies.

2. A method for obtaining quantitative information on average donor-acceptor distance changes between a first molecule and a second molecule using ensemble Förster resonance energy transfer (eFRET), the method comprising the steps of:

- obtaining a donor sample, wherein said donor sample comprising at least a donor and/or a donor-labelled copies of said first molecule;
- obtaining an acceptor sample comprising at least an acceptor and/or an acceptor-labelled copies of said second molecule; and
- obtaining at least a donor-acceptor-labelled sample comprising donor-labelled copies of said first molecule and acceptor-labelled copies of said second molecule;
- performing an eFRET measurement for the donor-acceptor-labelled sample under a first condition and a second condition to obtain for this samples a first eFRET result under the first condition and a second eFRET result under the second condition, wherein the eFRET measurements of the donor-acceptor-labelled sample are performed using multiple respective molecule copies;
- performing at least an eFRET measurement for the donor sample under at least one condition to obtain for this sample a first eFRET result and calculating a leakage coefficient ($c_{lk}$) from said donor sample;
- performing at least an eFRET measurement for the acceptor sample under at least one condition to obtain for this sample a first eFRET result and calculating a direct excitation coefficient ($c_{dirE}$);
- correcting the donor-acceptor-labelled eFRET results based on the leakage coefficient ($c_{lk}$) and the direct excitation coefficient ($c_{dirE}$);

wherein the first and/or the second eFRET results comprise respective measurement values of the

- emission of the acceptor after donor excitation ($F_{DA}^{(A)}$),
- emission of the acceptor after acceptor excitation ($F_{AA}^{(A)}$), and/or
- emission of the donor after donor excitation ($F_{DD}^{(D)}$),
- emission of the donor after acceptor excitation ($F_{DA}^{(D)}$)

wherein said leackage coefficient ($c_{lk}$) is determined based on the ratio of $F_{DA}^{(D)}$ and $F_{DD}^{(D)}$; and said direct excitation coefficient ($c_{dirE}$) is determined based on the ratio of $F_{DA}^{(A)}$ and $F_{AA}^{(A)}$;

- calculating a $\gamma$-correction factor based on the donor-acceptor-labelled sample(s), wherein the $\gamma$-correction factor is based on the negative ratio of i) the difference of $F_{DA}$ between the first and the second condition and ii) the difference of $F_{DD}$ of the donor-acceptor-labelled sample between the first and the second condition; or calculating the $\gamma$-correction factor using a photobleaching method in which the acceptor is partially photobleached which leads to a difference in FRET signal and therefore to a difference in donor signal;
- determining condition-specific eFRET efficiencies based on the corrected donor-acceptor-labelled eFRET results and by using the γ-correction factor; and
- determining quantitative information on average donor-acceptor distance changes between the first and second molecule under the first and the second condition using the condition-specific eFRET efficiencies.

3.  The method according to claim 1 or 2, wherein the step of obtaining at least the donor sample, the acceptor sample, and the donor-acceptor-labelled sample comprises the steps of:

    - obtaining at least a first, a second, and a third sample comprising multiple copies of the molecule each;
    - labelling molecule copies comprised in the first sample with the donor;
    - labelling molecule copies comprised in the second sample with the acceptor; and
    - labelling molecule copies comprised in the third sample with the donor and the acceptor,

    wherein the donor is preferably a fluorophore and wherein the acceptor is preferably a fluorophore.

4.  The method according to any of claims 1 to 3, wherein the donor has a fluorescence emission spectrum and the acceptor an excitation spectrum, and wherein the fluorescence emission spectrum of the donor overlaps with the excitation spectrum of the acceptor.

5.  The method according to any of claims 1 to 4, wherein the step of performing an eFRET measurement for said samples under the first and/or the second condition comprises for each eFRET measurement the steps of:

    - transmitting light having a wavelength within the excitation spectrum of the donor, preferably from a quasi monochromatic light source, preferably an LED, to the respective sample to excite the donor, and
    - transmitting light having a wavelength within the excitation spectrum of the acceptor, preferably from a quasi monochromatic light source, preferably an LED, to the respective sample to excite the acceptor.

6.  The method according to claim 5, wherein the light is transmitted to the respective sample in at least one illumination cycle, preferably 1-1000 illumination cycles, wherein an illumination cycle has preferably at least 1 light pulse, preferably 2 light pulses or even more between 10ms and 1s, preferably light pulses with light having alternately a wavelength within the excitation spectrum of the donor and the acceptor, respectively, wherein the measured emission of the acceptor and the donor is preferably averaged over the illumination cycles.

7.  The method according to any of claims 1 to 6, wherein said $F_{DA}$ and $F_{DD}$ for determining the leackage coefficient ($c_{lk}$) are obtained for the donor sample under the respective condition, and/or wherein said $F_{DA}$ and $F_{AA}$ for determining the direct excitation coefficient ($c_{dirE}$) are preferably obtained for the acceptor sample under the respective condition.

8.  The method according to any of claims 1 to 7, wherein the first and the second condition differ from each other

    i) by the presence of at least one further molecule, preferably a plurality of further molecules, in one of the two conditions, wherein the at least one further molecule, preferably the plurality of further molecule, preferably interacts with the molecule, and/or
    ii) in at least one, preferably one, parameter selected from the group consisting of temperature, pH, salt content, buffer composition, the addition of chaotropes, excipients, temperature and ionic strength.

9.  The method according to any of claims 1 to 8, wherein the method further comprises the steps of:

    - determining condition-specific correction factors using the respective first and second eFRET result under the first and second condition,
    - correcting the first and second eFRET results using the respective condition-specific correction factors,
    - determining an inter-condition correction factor using the condition-specific corrected first and second eFRET results, and
    - correcting the condition-specific corrected first and second eFRET results using the inter-condition correction factor.

10. The method according to claim 9, wherein the step of determining condition-specific correction factors using the respective first and second eFRET result comprises the steps of:

- determining a condition-specific leakage coefficient $c_{lk}$, and/or
- determining a condition-specific direct excitation coefficient $c_{dirE}$.

11. The method according to any of claims 7 to 10, wherein the step of correcting the first and second eFRET results comprises the step of:
determining a corrected $F_{DA}$ ($F_{DAcor}$) for the donor-acceptor-labelled sample by subtracting from $F_{DA}$ the $F_{DD}$ scaled by $c_{lk}$ and $F_{AA}$ *scaled by* $c_{dirE}$, wherein said $F_{DA}$, $F_{DD}$, and $F_{AA}$ are measured for the donor-acceptor-labelled sample under the respective condition.

12. The method according to any of claims 7 to 11, wherein the step of determining an inter-condition correction factor using the corrected first and second eFRET results comprises the step of:
determining the $\gamma$-correction factor based on the negative ratio of i) the difference of $F_{DAcor}$ between the first and the second condition and ii) the difference of $F_{DD}$ of the donor-acceptor-labelled sample between the first and the second condition.

13. The method according to any of claims 1 to 12, wherein the step of determining eFRET efficiencies based on the respective corrected first and second eFRET result comprises the steps of:

- determining respective $\gamma$-corrected $F_{DD}$ for the donor-acceptor-labelled sample under the respective condition, and
- determining eFRET efficiencies based on the ratio of i) the respective $F_{DAcor}$ and ii) the sum of the respective $F_{DAcor}$ and the respective $\gamma$-corrected $F_{DD}$.

14. The method according to any of claims 1 to 13, wherein the step of determining quantitative information on average donor-acceptor distance changes comprises the step of:
obtaining information on a donor-acceptor specific Förster radius, $R_0$, and on a donor-acceptor distance, r, under the first or the second condition.

15. A measurement system comprising a controller adapted for obtaining quantitative information on average donor-acceptor distance or on average donor-acceptor distance changes in accordance with any of claims 1 to 14, wherein said measurement system comprises:

- means for accommodating vessels, preferably a microwell plate or capillaries, wherein a vessel comprising a donor sample, an acceptor sample or a donor-acceptor-labelled sample;
- at least one light source, preferably a quasi monochromatic light source, preferably an LED;
- at least one light detector, preferably a PMT or siPM;
- preferably a filter, a dichroic mirror, a polychromic mirror, and/or an objective lens;
- means for performing an eFRET measurement using multiple respective molecule copies at least in the donor-acceptor-labelled sample; and
- control means adapted for

controlling the means for accommodating the vessel;
controlling the at least one light source for transmitting light from the light source to the vessel;
controlling the at least one light detector for detecting signals from the vessel; and
controlling said means for performing the eFRET measurement.

16. Use of a measurement system according to claim 15 to obtain quantitative information on average donor-acceptor distance changes within a molecule using ensemble Förster resonance energy transfer (eFRET) in accordance with any of claims 1 to 14.

17. A computer program in combination with a measurement system according to claim 15, said computer program comprising instructions which, when the program is executed by a computer, cause the measurement system to carry out the method according to any of claims 1 to 14.

18. A computer-readable data carrier comprising instructions which, when executed by a computer of measurement system according claim 15, cause the computer and the measurement system to carry out the method according to any of claims 1 to 14.

## Patentansprüche

1. Verfahren zur Gewinnung quantitativer Information über durchschnittliche Donor-Akzeptor-Abstandsänderungen in einem Molekül unter Verwendung von Ensemble-Förster-Resonanzenergietransfer (eFRET), wobei das Verfahren die folgenden Schritte aufweist:

- Gewinnen einer Donor-Probe, wobei die Donor-Probe mindestens einen Donor und/oder donor-markierte Kopien des Moleküls aufweist;
- Gewinnen einer Akzeptor-Probe, die mindestens einen Akzeptor und/oder akzeptor-markierte Kopien des Moleküls aufweist; und
- Gewinnen mindestens einer donor-akzeptor-markierten Probe, die donor-akzeptor-markierte Kopien des Moleküls aufweist;
- Durchführen einer eFRET-Messung für die donor-akzeptor-markierte Probe unter einer ersten Bedingung und einer zweiten Bedingung, um für diese Proben ein erstes eFRET-Ergebnis unter der ersten Bedingung und ein zweites eFRET-Ergebnis unter der zweiten Bedingung zu gewinnen, wobei die eFRET-Messungen der donor-akzeptor-markierten Probe unter Verwendung mehrerer jeweiliger Molekülkopien durchgeführt werden;
- Durchführen mindestens einer eFRET-Messung für die Donor-Probe unter mindestens einer Bedingung, um für diese Probe ein erstes eFRET-Ergebnis zu gewinnen, und Berechnen eines Leakage-Koeffizienten ($c_{lk}$) aus der Donor-Probe,
- Durchführen mindestens einer eFRET-Messung für die Akzeptor-Probe unter mindestens einer Bedingung, um für diese Probe ein erstes eFRET-Ergebnis zu gewinnen, und Berechnen eines Direkte-Anregung-Koeffizienten ($c_{dirE}$),
- Korrigieren der donor-akzeptor-markierten eFRET-Ergebnisse auf Basis des Leakage-Koeffizienten ($c_{lk}$) und des Direkte-Anregung-Koeffizienten ($c_{dirE}$);

wobei die ersten und/oder die zweiten eFRET-Ergebnisse jeweilige Messwerte der

- Emission des Akzeptors nach Donor-Anregung ($F_{DA}^{(A)}$),
- Emission des Akzeptors nach Akzeptor-Anregung ($F_{AA}^{(A)}$) und/oder
- Emission des Donors nach Donor-Anregung ($F_{DD}^{(D)}$),
- Emission des Donors nach Akzeptor-Anregung ($F_{DA}^{(D)}$)
aufweisen,

wobei der Leakage-Koeffizient ($c_{lk}$) auf Basis des Verhältnisses von $F_{DA}^{(D)}$ und $F_{DD}^{(D)}$ bestimmt wird; und der Direkte-Anregung-Koeffizient ($c_{dirE}$) auf Basis des Verhältnisses von $F_{DA}^{(A)}$ und $F_{AA}^{(A)}$ bestimmt wird;

- Berechnen eines $\gamma$-Korrekturfaktors auf Basis der donor-akzeptor-markierten Probe(n), wobei der $\gamma$-Korrekturfaktor basiert auf dem negativen Verhältnis von i) der Differenz von $F_{DA}$ zwischen der ersten und der zweiten Bedingung und ii) der Differenz von $F_{DD}$ der donor-akzeptor-markierten Probe zwischen der ersten und der zweiten Bedingung; oder
- Berechnen des $\gamma$-Korrekturfaktors unter Verwendung eines Photobleichungs-Verfahrens, bei dem der Akzeptor teilweise photogebleicht wird, was zu einer Differenz im FRET-Signal und damit zu einer Differenz im Donor-Signal führt;
- Bestimmen bedingungsspezifischer eFRET-Wirkungsgrade auf Basis der korrigierten donor-akzeptor-markierten eFRET-Ergebnisse und unter Verwendung des $\gamma$-Korrekturfaktors; und
- Bestimmen quantitativer Information über durchschnittliche Donor-Akzeptor-Abstandsänderungen in dem Molekül unter der ersten und der zweiten Bedingung unter Verwendung der bedingungsspezifischen eFRET-Wirkungsgrade.

2. Verfahren zur Gewinnung quantitativer Information über durchschnittliche Donor-Akzeptor-Abstandsänderungen zwischen einem ersten Molekül und einem zweiten Molekül unter Verwendung von Ensemble-Förster-Resonanzenergietransfer (eFRET), wobei das Verfahren die folgenden Schritte aufweist:

- Gewinnen einer Donor-Probe, wobei die Donor-Probe mindestens einen Donor und/oder donor-markierte Kopien des ersten Moleküls aufweist;

- Gewinnen einer Akzeptor-Probe, die mindestens einen Akzeptor und/oder akzeptor-markierte Kopien des zweiten Moleküls aufweist; und
- Gewinnen mindestens einer donor-akzeptor-markierten Probe, die donor-markierte Kopien des ersten Moleküls und akzeptor-markierte Kopien des zweiten Moleküls aufweist;
- Durchführen einer eFRET-Messung für die donor-akzeptor-markierte Probe unter einer ersten Bedingung und einer zweiten Bedingung, um für diese Proben ein erstes eFRET-Ergebnis unter der ersten Bedingung und ein zweites eFRET-Ergebnis unter der zweiten Bedingung zu gewinnen, wobei die eFRET-Messungen der donor-akzeptor-markierten Probe unter Verwendung mehrerer jeweiliger Molekülkopien durchgeführt werden;
- Durchführen mindestens einer eFRET-Messung für die Donor-Probe unter mindestens einer Bedingung, um für diese Probe ein erstes eFRET-Ergebnis zu gewinnen, und Berechnen eines Leakage-Koeffizienten ($c_{lk}$) aus der Donor-Probe;
- Durchführen mindestens einer eFRET-Messung für die Akzeptor-Probe unter mindestens einer Bedingung, um für diese Probe ein erstes eFRET-Ergebnis zu gewinnen, und Berechnen eines Direkte-Anregung-Koeffizienten ($c_{dirE}$);

Korrigieren der donor-akzeptor-markierten eFRET-Ergebnisse auf Basis des Leakage-Koeffizienten ($c_{lk}$) und des Direkte-Anregung-Koeffizienten ($c_{dirE}$);
wobei die ersten und/oder die zweiten eFRET-Ergebnisse jeweilige Messwerte der

- Emission des Akzeptors nach Donor-Anregung ($F_{DA}^{(A)}$),
- Emission des Akzeptors nach Akzeptor-Anregung ($F_{AA}^{(A)}$) und/oder
- Emission des Donors nach Donor-Anregung ($F_{DD}^{(D)}$),
- Emission des Donors nach Akzeptor-Anregung ($F_{DA}^{(D)}$)
aufweisen,

wobei der Leakage-Koeffizient ($c_{lk}$) auf Basis des Verhältnisses von $F_{DA}^{(D)}$ und $F_{DD}^{(D)}$ bestimmt wird; und der Direkte-Anregung-Koeffizient ($c_{dirE}$) auf Basis des Verhältnisses von $F_{DA}^{(A)}$ und $F_{AA}^{(A)}$ bestimmt wird;

- Berechnen eines $\gamma$-Korrekturfaktors auf Basis der donor-akzeptor-markierten Probe(n), wobei der $\gamma$-Korrekturfaktor basiert auf dem negativen Verhältnis von i) der Differenz von $F_{DA}$ zwischen der ersten und der zweiten Bedingung und ii) der Differenz von $F_{DD}$ der donor-akzeptor-markierten Probe zwischen der ersten und der zweiten Bedingung; oder
- Berechnen des $\gamma$-Korrekturfaktors unter Verwendung eines Photobleichungs-Verfahrens, bei dem der Akzeptor teilweise photogebleicht wird, was zu einer Differenz im FRET-Signal und damit zu einer Differenz im Donor-Signal führt;
- Bestimmen bedingungsspezifischer eFRET-Wirkungsgrade auf Basis der korrigierten donor-akzeptor-markierten eFRET-Ergebnisse und unter Verwendung des $\gamma$-Korrekturfaktors; und
- Bestimmen quantitativer Information über durchschnittliche Donor-Akzeptor-Abstandsänderungen zwischen dem ersten und zweiten Molekül unter der ersten und der zweiten bedingung unter Verwendung der bedingungsspezifischen eFRET-Wirkungsgrade.

3.  Verfahren nach Anspruch 1 oder 2, wobei der Schritt Gewinnen mindestens der Donor-Probe, der Akzeptor-Probe und der donor-akzeptor-markierten Probe die Schritte aufweist:

- Gewinnen mindestens einer ersten, einer zweiten und einer dritten Probe, die jeweils mehrere Kopien des Moleküls aufweisen;
- Markieren von Molekülkopien, die in der ersten Probe enthalten sind, mit dem Donor;
- Markieren von Molekülkopien, die in der zweiten Probe enthalten sind, mit dem Akzeptor; und
- Markieren von Molekülkopien, die in der dritten Probe enthalten sind, mit dem Donor und dem Akzeptor,

wobei der Donor vorzugsweise ein Fluorophor ist, und wobei der Akzeptor vorzugsweise ein Fluorophor ist.

4.  Verfahren nach einem der Ansprüche 1 bis 3, wobei der Donor ein Fluoreszenzemissionsspektrum und der Akzeptor ein Anregungsspektrum hat, und wobei das Fluoreszenzemissionsspektrum des Donors sich mit dem Anregungsspektrum des Akzeptors überlappt.

5.  Verfahren nach einem der Ansprüche 1 bis 4, wobei für jede eFRET-Messung der Schritt Durchführen einer eFRET-Messung für die Proben unter der ersten und/oder der zweiten Bedingung die Schritte aufweist:

- Senden von Licht, das eine Wellenlänge in dem Anregungsspektrum des Donors hat, vorzugsweise aus einer quasimonochromatischen Lichtquelle, vorzugsweise einer LED, zu der jeweiligen Probe, um den Donor anzuregen, und

- Senden von Licht, das eine Wellenlänge in dem Anregungsspektrum des Akzeptors hat, vorzugsweise aus einer quasimonochromatischen Lichtquelle, vorzugsweise einer LED, zu der jeweiligen Probe, um den Akzeptor anzuregen.

6. Verfahren nach Anspruch 5, wobei das Licht in mindestens einem einzigen Belichtungszyklus, vorzugsweise 1-1000 Belichtungszyklen, zu der jeweiligen Probe gesendet wird, wobei ein Belichtungszyklus vorzugsweise mindestens 1 Lichtpuls, vorzugsweise 2 Lichtpulse oder noch mehr zwischen 10ms und 1s hat, vorzugsweise Lichtpulse mit Licht, das abwechselnd eine Wellenlänge in dem Anregungsspektrum des Donors bzw. des Akzeptors hat, wobei die gemessene Emission des Akzeptors und des Donors vorzugsweise über die Belichtungszyklen gemittelt wird.

7. Verfahren nach einem der Ansprüche 1 bis 6,

wobei $F_{DA}$ und $F_{DD}$ zur Bestimmung des Leakage-Koeffizienten ($c_{lk}$) für die Donor-Probe unter der jeweiligen Bedingung gewonnen werden, und/oder

wobei $F_{DA}$ und $F_{AA}$ zur Bestimmung des Direkte-Anregung-Koeffizienten ($c_{dirE}$) für die Akzeptor-Probe vorzugsweise unter der jeweiligen Bedingung gewonnen werden.

8. Verfahren nach einem der Ansprüche 1 bis 7, wobei die erste und die zweite Bedingung sich voneinander unterscheiden

i) durch die Anwesenheit mindestens eines weiteren Moleküls, vorzugsweise einer Mehrzahl weiterer Moleküle, in einer der zwei Bedingungen, wobei das mindestens eine weitere Molekül, vorzugsweise die Mehrzahl weiterer Moleküle, vorzugsweise mit dem Molekül wechselwirkt, und/oder

ii) in mindestens einem, vorzugsweise einem einzigen, aus der folgenden Gruppe ausgewählten Parameter: Temperatur, pH, Salzgehalt, Pufferzusammensetzung, die Zugabe von Chaotropen, Trägermitteln, Temperatur und Ionenstärke.

9. Verfahren nach einem der Ansprüche 1 bis 8, wobei das Verfahren desweiteren die Schritte aufweist:

- Bestimmen bedingungsspezifischer Korrekturfaktoren unter Verwendung des jeweiligen ersten und zweiten eFRET-Ergebnisses unter der ersten und zweiten Bedingung,

- Korrigieren der ersten und zweiten eFRET-Ergebnisse unter Verwendung der jeweiligen bedingungsspezifischen Korrekturfaktoren,

- Bestimmen eines inter-bedingungs-Korrekturfaktors unter Verwendung der bedingungsspezifischen korrigierten ersten und zweiten eFRET-Ergebnisse, und

- Korrigieren der bedingungsspezifischen korrigierten ersten und zweiten eFRET-Ergebnisse unter Verwendung des inter-bedingungs-Korrekturfaktors.

10. Verfahren nach Anspruch 9, wobei der Schritt Bestimmen bedingungsspezifischer Korrekturfaktoren unter Verwendung des jeweiligen ersten und zweiten eFRET-Ergebnisses die Schritte aufweist:

- Bestimmen eines bedingungsspezifischen Leakage-Koeffizienten $c_{lk}$, und/oder

- Bestimmen eines bedingungsspezifischen Direkte-Anregung-Koeffizienten $c_{dirE}$.

11. Verfahren nach einem der Ansprüche 7 bis 10, wobei der Schritt Korrigieren der ersten und zweiten eFRET-Ergebnisse den Schritt aufweist:

Bestimmen eines korrigierten $F_{DA}$ ($F_{DAcor}$) für die donor-akzeptor-markierte Probe durch Subtrahieren von $F_{DA}$ den mit $c_{lk}$ skalierten $F_{DD}$ und den mit $c_{dirE}$ skalierten $F_{AA}$, wobei $F_{DA}$, $F_{DD}$ und $F_{AA}$ für die donor-akzeptor-markierte Probe unter der jeweiligen Bedingung gemessen sind.

12. Verfahren nach einem der Ansprüche 7 bis 11, wobei der Schritt Bestimmen eines inter-bedingungs-Korrekturfaktors unter Verwendung der korrigierten ersten und zweiten eFRET-Ergebnisse den Schritt aufweist:

Bestimmen des $\gamma$-Korrekturfaktors auf Basis des negativen Verhältnisses von i) der Differenz von $F_{DAcor}$ zwischen der ersten und der zweiten Bedingung und ii) der Differenz von $F_{DD}$ der donor-akzeptor-markierten Probe zwischen der ersten und der zweiten Bedingung.

**13.** Verfahren nach einem der Ansprüche 1 bis 12, wobei der Schritt Bestimmen von eFRET-Wirkungsgraden auf Basis des jeweiligen korrigierten ersten und zweiten eFRET-Ergebnisses die Schritte aufweist:

- Bestimmen jeweiliger $\gamma$-korrigierter $F_{DD}$ für die donor-akzeptor-markierte Probe unter der jeweiligen Bedingung, und
- Bestimmen von eFRET-Wirkungsgraden auf Basis des Verhältnisses von i) des jeweiligen $F_{DAcor}$ und ii) der Summe des jeweiligen $F_{DAcor}$ und des jeweiligen $\gamma$-korrigierten $F_{DD}$.

**14.** Verfahren nach einem der Ansprüche 1 bis 13, wobei der Schritt Bestimmen quantitativer Information über durchschnittliche Donor-Akzeptor-Abstandsänderungen den Schritt aufweist:
Gewinnung von Information über einen donor-akzeptor-spezifischen Förster-Radius, $R_0$, und über einen Donor-Akzeptor-Abstand, r, unter der ersten oder der zweiten Bedingung.

**15.** Messungssystem aufweisend eine angepasste Steuerung zur Gewinnung quantitativer Information über einen durchschnittlichen Donor-Akzeptor-Abstand oder über durchschnittliche Donor-Akzeptor-Abstandsänderungen gemäß einem der Ansprüche 1 bis 14, wobei das Messungssystem aufweist:

- Einrichtung zur Aufnahme von Gefäßen, vorzugsweise einer Mikrotiterplatte oder Kapillargefäßen, wobei ein Gefäß eine Donor-Probe, eine Akzeptor-Probe oder eine donor-akzeptor-markierte Probe aufweist;
- mindestens eine Lichtquelle, vorzugsweise eine quasimonochromatische Lichtquelle, vorzugsweise eine LED;
- mindestens einen Lichtdetektor, vorzugsweise einen PMT oder siPM;
- vorzugsweise ein Filter, einen dichroitischen Spiegel, einen polychromen Spiegel und/oder eine Objektivlinse;
- Einrichtung zum Durchführen einer eFRET-Messung unter Verwendung mehrerer jeweiliger Molekülkopien zumindest in der donor-akzeptor-markierten Probe; und
- angepasste Steuereinrichtung zur
Steuerung der Einrichtung zur Aufnahme des Gefäßes;
Steuerung der mindestens einen Lichtquelle zum Senden von Licht aus der Lichtquelle zu dem Gefäß;
Steuerung des mindestens einen Lichtdetektors zum Detektieren von Signalen aus dem Gefäß; und
Steuerung der Einrichtung zur Durchführung der eFRET-Messung.

**16.** Verwendung eines Messungssystems nach Anspruch 15, um quantitative Information über durchschnittliche Donor-Akzeptor-Abstandsänderungen in einem Molekül unter Verwendung von Ensemble Förster Resonanzenergietransfer (eFRET) nach einem der Ansprüche 1 bis 14 zu gewinnen.

**17.** Computerprogramm in Kombination mit einem Messungssystem nach Anspruch 15, wobei das Computerprogramm Befehle aufweist, die, wenn das Programm von einem Computer ausgeführt wird, das Messungssystem veranlassen, das Verfahren nach einem der Ansprüche 1 bis 14 durchzuführen.

**18.** Computerlesbarer Datenträger aufweisend Befehle, die, wenn sie von einem Computer des Messungssystems nach Anspruch 15 ausgeführt werden, den Computer und das Messungssystem veranlassen, das Verfahren nach einem der Ansprüche 1 bis 14 durchzuführen.

**Revendications**

**1.** Procédé d'obtention d'informations quantitatives sur des changements de distance donneur-accepteur moyenne au sein d'une molécule à l'aide du transfert d'énergie de résonance Förster d'ensemble (eFRET), le procédé comprenant les étapes de :

- obtention d'un échantillon donneur, ledit échantillon donneur comprenant au moins un donneur et/ou des copies marquées par donneur de ladite molécule ;
- obtention d'un échantillon accepteur comprenant au moins un accepteur et/ou des copies marquées par accepteur de ladite molécule ; et
- obtention d'au moins un échantillon marqué par donneur-accepteur comprenant des copies marquées par donneur-accepteur de ladite molécule ;
- réalisation d'une mesure eFRET pour l'échantillon marqué par donneur-accepteur dans une première condition et une deuxième condition pour obtenir, pour ces échantillons, un premier résultat eFRET dans la première condition et un deuxième résultat eFRET dans la deuxième condition, les mesures eFRET de l'échantillon marqué par donneur-accepteur étant effectuées en utilisant plusieurs copies de molécule respectives ;

- réalisation d'au moins une mesure eFRET pour l'échantillon donneur dans au moins une condition pour obtenir, pour cet échantillon, un premier résultat eFRET et calcul d'un coefficient de fuite ($c_{lk}$) à partir dudit échantillon donneur ;
- réalisation d'au moins une mesure eFRET pour l'échantillon accepteur dans au moins une condition pour obtenir, pour cet échantillon, un premier résultat eFRET et calcul d'un coefficient d'excitation directe ($c_{dirE}$) ;
- correction des résultats eFRET marqués par donneur-accepteur en fonction du coefficient de fuite ($c_{lk}$) et du coefficient d'excitation directe ($c_{dirE}$) ;

dans lequel les premiers et/ou deuxièmes résultats eFRET comprennent des valeurs de mesure respectives de

- émission de l'accepteur après excitation du donneur ($F_{DA}^{(A)}$),
- émission de l'accepteur après excitation de l'accepteur ($F_{AA}^{(A)}$), et/ou
- émission du donneur après excitation du donneur ($FDD^{(D)}$),
- émission du donneur après excitation de l'accepteur ($F_{DA}^{(D)}$) dans lequel ledit coefficient de fuite ($c_{lk}$) est déterminé sur la base du rapport de $F_{DA}^{(D)}$ et $F_{DD}^{(D)}$ ; et ledit coefficient d'excitation directe ($c_{dirE}$) est déterminé sur la base du rapport de $F_{DA}^{(D)}$ et $F_{AA}^{(A)}$ ;

- calcul d'un facteur de correction $\gamma$ sur la base des un ou plusieurs échantillons marqués par donneur-accepteur, le facteur de correction $\gamma$ étant basé sur le rapport négatif de i) la différence de $F_{DA}$ entre la première et la deuxième condition et ii) la différence de $F_{DD}$ de l'échantillon marqué par donneur-accepteur entre la première et la deuxième condition ; ou
calcul du facteur de correction $\gamma$ à l'aide d'un procédé de photoblanchiment dans lequel l'accepteur est partiellement photoblanchi, ce qui conduit à une différence de signal FRET et, par conséquent, à une différence de signal de donneur ;
- détermination des efficacités eFRET spécifiques à une condition sur la base des résultats eFRET marqués par donneur-accepteur et à l'aide du facteur de correction $\gamma$ ; et
- détermination d'informations quantitatives sur les changements de distance donneur-accepteur moyenne au sein de la molécule dans la première et la deuxième condition à l'aide des efficacités eFRET spécifiques à la condition.

2. Procédé d'obtention d'informations quantitatives sur des changements de distance donneur-accepteur moyenne entre une première molécule et une deuxième molécule à l'aide du transfert d'énergie de résonance Förster d'ensemble (eFRET), le procédé comprenant les étapes de :

- obtention d'un échantillon de donneur, ledit échantillon de donneur comprenant au moins un donneur et/ou des copies marquées par donneur de ladite première molécule ;
- obtention d'un échantillon d'accepteur comprenant au moins un accepteur et/ou des copies marquées par un accepteur de ladite deuxième molécule ; et
- obtention d'au moins un échantillon marqué par donneur-accepteur comprenant des copies marquées par donneur de ladite première molécule et des copies marquées par accepteur de ladite deuxième molécule ;
- réalisation d'une mesure eFRET pour l'échantillon marqué par donneur-accepteur dans une première condition et une deuxième condition pour obtenir, pour ces échantillons, un premier résultat eFRET dans la première condition et un deuxième résultat eFRET dans la deuxième condition, les mesures eFRET de l'échantillon marqué par donneur-accepteur étant effectuées en utilisant plusieurs copies de molécule respectives ;
- réalisation d'au moins une mesure eFRET pour l'échantillon donneur dans au moins une condition pour obtenir, pour cet échantillon, un premier résultat eFRET et calcul d'un coefficient de fuite ($c_{lk}$) à partir dudit échantillon donneur ;
- réalisation d'au moins une mesure eFRET pour l'échantillon accepteur dans au moins une condition pour obtenir, pour cet échantillon, un premier résultat eFRET et calcul d'un coefficient d'excitation directe ($c_{dirE}$) ;
- correction des résultats eFRET marqués par donneur-accepteur en fonction du coefficient de fuite ($c_{lk}$) et du coefficient d'excitation directe ($c_{dirE}$) ; dans lequel les premiers et/ou deuxièmes résultats eFRET comprennent des valeurs de mesure respectives de

- émission de l'accepteur après excitation du donneur ($F_{DA}^{(A)}$),
- émission de l'accepteur après excitation de l'accepteur ($F_{AA}^{(A)}$), et/ou
- émission du donneur après excitation du donneur ($F_{DD}^{(D)}$),
- émission du donneur après excitation de l'accepteur ($F_{DA}^{(D)}$) dans lequel ledit coefficient de fuite ($c_{lk}$) est déterminé sur la base du rapport de $F_{DA}^{(D)}$ et $F_{DD}^{(D)}$ ; et ledit coefficient d'excitation directe ($c_{dirE}$) est déterminé sur la base du rapport de $F_{DA}^{(D)}$ et $F_{AA}^{(A)}$ ;

- calcul d'un facteur de correction $\gamma$ sur la base des un ou plusieurs échantillons marqués par donneur-accepteur, le facteur de correction $\gamma$ étant basé sur le rapport négatif de i) la différence de $F_{DA}$ entre la première et la deuxième condition et ii) la différence de $F_{DD}$ de l'échantillon marqué par donneur-accepteur entre la première et la deuxième condition ; ou

calcul du facteur de correction $\gamma$ à l'aide d'un procédé de photoblanchiment dans lequel l'accepteur est partiellement photoblanchi, ce qui conduit à une différence de signal FRET et, par conséquent, à une différence de signal de donneur ;

- détermination des efficacités eFRET spécifiques à une condition sur la base des résultats eFRET corrigés marqués par donneur-accepteur et à l'aide du facteur de correction $\gamma$ ; et

- détermination d'informations quantitatives sur les changements de distance donneur-accepteur moyenne entre la première et la deuxième molécule dans la première et la deuxième condition à l'aide des efficacités eFRET spécifiques à la condition.

**3.** Procédé selon la revendication 1 ou 2, dans lequel l'étape d'obtention d'au moins l'échantillon donneur, l'échantillon accepteur et l'échantillon marqué par donneur-accepteur comprend les étapes de :

- obtention d'au moins un premier, un deuxième et un troisième échantillon comprenant chacun des copies multiples de la molécule ;
- marquage des copies de molécules comprises dans le premier échantillon avec le donneur ;
- marquage des copies de molécules comprises dans le deuxième échantillon avec l'accepteur ; et
- marquage des copies de molécules comprises dans le troisième échantillon avec le donneur et l'accepteur,

dans lequel le donneur est de préférence un fluorophore et dans lequel l'accepteur est de préférence un fluorophore.

**4.** Procédé selon l'une quelconque des revendications 1 à 3, dans lequel le donneur a un spectre d'émission de fluorescence et l'accepteur un spectre d'excitation, et dans lequel le spectre d'émission de fluorescence du donneur chevauche le spectre d'excitation de l'accepteur.

**5.** Procédé selon l'une quelconque des revendications 1 à 4, dans lequel l'étape de réalisation d'une mesure eFRET pour lesdits échantillons dans la première et/ou la deuxième condition comprend, pour chaque mesure eFRET, les étapes de :

- transmission d'une lumière ayant une longueur d'onde dans le spectre d'excitation du donneur, de préférence à partir d'une source de lumière quasi-monochromatique, de préférence une DEL, vers l'échantillon respectif pour exciter le donneur, et
- transmission d'une lumière ayant une longueur d'onde dans le spectre d'excitation de l'accepteur, de préférence à partir d'une source de lumière quasi-monochromatique, de préférence une DEL, vers l'échantillon respectif pour exciter l'accepteur.

**6.** Procédé selon la revendication 5, dans lequel la lumière est transmise à l'échantillon respectif dans au moins un cycle d'éclairage, de préférence 1 à 1 000 cycles d'éclairage, dans lequel un cycle d'éclairage a de préférence au moins 1 impulsion lumineuse, de préférence 2 impulsions lumineuses ou encore plus préférablement entre 10 ms et 1 s, de préférence des impulsions lumineuses avec une lumière ayant alternativement une longueur d'onde dans le spectre d'excitation du donneur et de l'accepteur, respectivement, l'émission mesurée de l'accepteur et du donneur étant de préférence moyennée sur les cycles d'éclairage.

**7.** Procédé selon l'une quelconque des revendications 1 à 6, dans lequel lesdits $F_{DA}$ et $F_{DD}$ pour déterminer le coefficient de fuite ($c_{lk}$) sont obtenus pour l'échantillon du donneur dans les conditions respectives, et/ou dans lequel lesdits $F_{DA}$ et $F_{AA}$ pour déterminer le coefficient d'excitation directe ($c_{dirE}$) sont de préférence obtenus pour l'échantillon accepteur dans la condition respective.

**8.** Procédé selon l'une quelconque des revendications 1 à 7, dans lequel les première et deuxième conditions diffèrent l'une de l'autre

i) par la présence d'au moins une autre molécule, de préférence d'une pluralité d'autres molécules, dans l'une des deux conditions, l'au moins une autre molécule, de préférence la pluralité d'autres molécules, interagissant de préférence avec la molécule, et/ou
ii) par au moins un, de préférence un, paramètre choisi dans le groupe constitué de la température, du pH, de la teneur en sel, de la composition tampon, de l'ajout de chaotropes, des excipients, de la température et de la force ionique.

**9.** Procédé selon l'une quelconque des revendications 1 à 8, le procédé comprenant en outre les étapes de :

- détermination de facteurs de correction spécifiques à une condition à l'aide des premiers et deuxièmes résultats eFRET respectifs dans la première et la deuxième condition,
- correction des premier et deuxième résultats eFRET à l'aide des facteurs de correction spécifiques à une condition respectifs,
- détermination d'un facteur de correction inter-conditions à l'aide des premier et deuxième résultats eFRET corrigés spécifiques à une condition, et
- correction des premier et deuxième résultats eFRET corrigés spécifiques à une condition à l'aide du facteur de correction inter-conditions.

**10.** Procédé selon la revendication 9, dans lequel l'étape de détermination de facteurs de correction spécifiques à une condition à l'aide des premier et deuxième résultats eFRET respectifs comprend les étapes de :

- détermination d'un coefficient de fuite spécifique à une condition $c_{lk}$, et/ou
- détermination d'un coefficient d'excitation directe spécifique à une condition $c_{dirE.}$

**11.** Procédé selon l'une quelconque des revendications 7 à 10, dans lequel l'étape de correction des premier et deuxième résultats eFRET comprend l'étape de :
détermination d'un $F_{DA}$ corrigé ($F_{DAcor}$) pour l'échantillon marqué par donneur-accepteur par soustraction à $F_{DA}$ le $F_{DD}$ mis à l'échelle par $c_{lk}$ et $F_{AA}$ mis à l'échelle par $c_{dirE}$, dans lequel lesdits $F_{DA}$, $F_{DD}$ et $F_{AA}$ sont mesurés pour l'échantillon marqué par donneur-accepteur dans la condition respective.

**12.** Procédé selon l'une quelconque des revendications 7 à 11, dans lequel l'étape de détermination d'un facteur de correction inter-conditions à l'aide des premier et deuxième résultats eFRET corrigés comprend l'étape de :
détermination du facteur de correction $\gamma$ sur la base du rapport négatif de i) la différence de $F_{DAcor}$ entre la première et la deuxième condition et ii) la différence de $F_{DD}$ de l'échantillon marqué par donneur-accepteur entre la première et la deuxième condition.

**13.** Procédé selon l'une quelconque des revendications 1 à 12, dans lequel l'étape de détermination des efficacités eFRET sur la base des premiers et deuxièmes résultats eFRET corrigés respectifs comprend les étapes suivantes :

- détermination du $F_{DD}$ corrigé par $\gamma$ pour l'échantillon marqué par donneur-accepteur dans la condition respective, et
- détermination des efficacités eFRET sur la base du rapport i) du $F_{DAcor}$ respectif et ii) de la somme du $F_{DAcor}$ respectif et du $F_{DD}$ corrigé par $\gamma$ respectif.

**14.** Procédé selon l'une quelconque des revendications 1 à 13, dans lequel l'étape de détermination d'informations quantitatives sur les changements de distance donneur-accepteur moyenne comprend l'étape de :
obtention d'informations sur un rayon Förster spécifique au donneur-accepteur, $R_0$, et sur une distance donneur-accepteur, r, dans la première ou la deuxième condition.

**15.** Système de mesure comprenant un dispositif de commande adapté pour obtenir des informations quantitatives sur la distance donneur-accepteur moyenne ou sur les changements de distance donneur-accepteur moyenne selon l'une quelconque des revendications 1 à 14, ledit système de mesure comprenant :

- des moyens pour recevoir des récipients, de préférence une plaque à micropuits ou des capillaires, un récipient comprenant un échantillon donneur, un échantillon accepteur ou un échantillon marqué par donneur-accepteur ;
- au moins une source de lumière, de préférence une source de lumière quasi-monochromatique, de préférence une DEL ;
- au moins un détecteur de lumière, de préférence un PMT ou un siPM ;
- de préférence un filtre, un miroir dichroïque, un miroir polychrome et/ou un objectif ;
- des moyens pour effectuer une mesure eFRET en utilisant des copies de molécules respectives multiples au moins dans l'échantillon marqué par donneur-accepteur ; et
- des moyens de commande adaptés pour

commander les moyens pour recevoir le récipient ;
commander l'au moins une source de lumière pour transmettre la lumière de la source de lumière au récipient ;
commander l'au moins un détecteur de lumière pour détecter des signaux provenant du récipient ; et
commander lesdits moyens pour effectuer la mesure eFRET.

16. Utilisation d'un système de mesure selon la revendication 15 pour obtenir des informations quantitatives sur les changements de distance donneur-accepteur moyenne au sein d'une molécule à l'aide du transfert d'énergie de résonance de Förster d'ensemble (eFRET) selon l'une quelconque des revendications 1 à 14.

17. Programme informatique en combinaison avec un système de mesure selon la revendication 15, ledit programme informatique comprenant des instructions qui, lorsque le programme est exécuté par un ordinateur, amènent le système de mesure à réaliser le procédé selon l'une quelconque des revendications 1 à 14.

18. Support de données lisible par ordinateur comprenant des instructions qui, lorsqu'elles sont exécutées par un ordinateur du système de mesure selon la revendication 15, amènent l'ordinateur et le système de mesure à réaliser le procédé selon l'une quelconque des revendications 1 à 14.

**Fig. 1**

**Fig. 2**

Fig. 3

**Fig. 4**

**Fig. 4-2**

# Fig. 5

Fig. 6

Fig. 7

Fig. 8

Fig. 9

**Fig. 10**

# Fig. 11

# Fig. 12

protein

3 samples of differently labelled proteins

measuring FRET signals of donor sample, acceptor sample, donor-acceptor-labelled sample, with and without ligand

| correction factor "leakage" | correction factor "direct excitation" | raw data |

correction of spectral artefacts ("leakage", "direct excitation"). Calculation of "gamma" and calculation of FRET effciency

ratio of FRET efficiencies with and without ligand

using reference values to calculate abolute distances

absolute distances or distance changes

# Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **EGGELING** et al. *PNAS,* 1998, vol. 95 (4), 1556-1561
- **NIR** et al. *J PHYS CHEM B,* 2006, vol. 110 (44), 22103-22124
- **HOHLBEIN** et al. *Chemical Society Reviews,* 2014, vol. 43 (4), 1156-1171
- **HOHLBEIN** et al. *CHEM SOC REV,* 2014, vol. 43 (4), 1156-1171